# EUROPEAN PATENT SPECIFICATION

(11) **EP 0 247 266 B1**
(45) Date of publication and mention of the grant of the patent: **10.03.1993**
(21) Application number: 86310011.1
(22) Date of filing: 22.12.1986
(51) Int. Cl.: C07D 451/04, C07D 451/12, C07D 451/14, C07D 453/02, C07D 209/08, A61K 31/46, A61K 31/445

(54) **Indole derivatives having an azabicyclic side chain, process for their preparation, intermediates, and pharmaceutical compositions**
Indolderivate mit einer azabicyclischen Seitenkette, Verfahren zu ihrer Herstellung, Zwischenprodukte und pharmazeutische Zusammensetzungen
Dérivés d'indole ayant une chaîne latérale azabicyclique, procédé pour leur préparation, produits intermédiaires et compositions pharmaceutiques

(30) Priority: 07.01.1986 GB 8600262; 07.11.1986 GB 8626632
(43) Date of publication of application: 02.12.1987
(73) Proprietor: BEECHAM GROUP PLC, Brentford, Middlesex TW8 9BD (GB)
(72) Inventor: King, Francis David Beecham Pharmaceuticals, The Pinnacles Harlow Essex, CM19 5AD (GB); Joiner, Karen Anne Beecham Pharmaceuticals, The Pinnacles Harlow Essex, CM19 5AD (GB)
(74) Representative: Tocher, Pauline

(56) References cited:
- EP-A- 0 115 933
- EP-A- 0 158 265
- EP-A- 0 200 444
- WO-A-84/00166
- WO-A-84/03281
- WO-A-85/01048
- WO-A-85/02847
- GB-A- 2 100 259

## Description

This invention relates to novel compounds having useful pharmacological properties, to pharmaceutical compositions containing them, to a process and intermediates for their preparation, and to their use as pharmaceuticals.

GB 2100259A and 2125398A, and EP-A-158265 describe benzoates and benzamides having an azabicyclic side chain and possessing 5-HT antagonist activity.

A class of novel, structurally distinct compounds has now been discovered. These compounds have 5-HT M-receptor antagonist activity, anti-emetic activity and/or gastric motility enhancing activity.

WO 85/01048 discloses certain indole carboxylic acid esters and amides having an azabicyclic side chain and possessing 5-HT antagonist activity.

Accordingly, the present invention provides a compound of formula (I), or a pharmaceutically acceptable salt thereof: 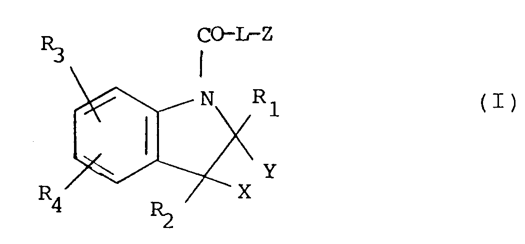
wherein
L is NH or O;
X and Y are independently selected from hydrogen or C₁₋₄ alkyl, or together are a bond;
R₁ and R₂ are independently selected from hydrogen, C₁₋₆ alkyl, C₂₋₆ alkenyl-C₁₋₄ alkyl, or together are C₂₋₄ polymethylene;
R₃ and R₄ are independently selected from hydrogen, halogen, CF₃, C₁₋₆ alkyl, C₁₋₆ alkoxy, C₁₋₆ alkylthio, C₁₋₇ acyl, C₁₋₇ acylamino, C₁₋₆ alkylsulphonylamino, N-(C₁₋₆ alkylsulphonyl)-N-C₁₋₄ alkylamino, C₁₋₆ alkylsulphinyl, hydroxy, nitro or amino, aminocarbonyl, aminosulphonyl, aminosulphonylamino or N-(aminosulphonyl)-C₁₋₄ alkylamino optionally N-substituted by one or two groups selected from C₁₋₆ alkyl, C₃₋₈ cycloalkyl, C₃₋₈ cycloalkyl C₁₋₄ alkyl, phenyl or phenyl C₁₋₄ alkyl groups or optionally N-disubstituted by C₄₋₅ polymethylene;
Z is a group of formula (a), (b) or (c) 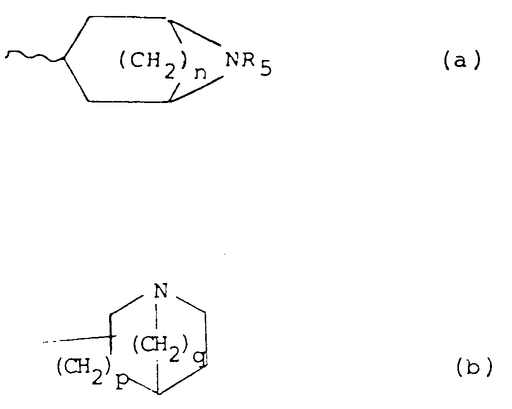 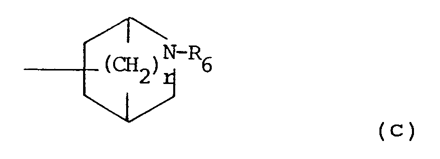
wherein n is 2 or 3; p is 1 or 2; q is 1 to 3; r is 1 to 3; and
R₅ or R₆ is C₁₋₇ alkyl, C₃₋₈ cycloalkyl, C₃₋₈ cycloalkyl-C₁₋₂ alkyl or C₂₋₇ alkenyl-C₁₋₄ alkyl; with the proviso that the compound of formula (I) is other than 2,3-dihydroindole-1-carboxylic acid 1-azabicyclo [2.2.2]oct-3-yl ester..

Preferably L is NH.

Suitable values for X and Y include hydrogen, methyl, ethyl, n- and iso-propyl; or together are a bond.

Often X and Y are both hydrogen.

Suitable values for R₁ or R₂ include hydrogen, methyl, ethyl, n- and iso-propyl; prop-2-enyl, but-2-enyl, but-3-enyl, 1-methylenepropyl and 1-methylprop-2-yl in their E and Z forms where stereoisomerism exists; or R₁ and R₂ together are as defined in formula (I). Often R₁ and R₂ are both hydrogen.

Values for R₃ and/or R₄ include hydrogen, fluoro, chloro, bromo, CF₃, methyl, ethyl, methoxy, ethoxy, methylthio, ethylthio, acetyl, propionyl, acetylamino, methylsulphonylamino, methylsulphinyl, hydroxy, nitro; and amino, aminocarbonyl, aminosulphonyl, aminosulphonylamino or N-(aminosulphonyl)-methylamino any of which may be optionally substituted by one or two methyl groups or by a cyclopentyl or cyclohexyl group or disubstituted by C₄ or C₅ polymethylene; R₃ is often hydrogen and R₄ is hydrogen or a 5-substituent, such as halo or methoxy.

Preferably n is 2 or 3 and p, q and r are 1 or 2.

Examples of R₅/R₆ when C₁₋₇ alkyl include as groups of interest C₁₋₃ alkyl such as methyl, ethyl and n- and iso-propyl. Within C₁₋₇ alkyl, C₄₋₇ alkyl are also of interest, especially those of the formula (CH₂)uR₉ wherein u is 1 or 2 and R₉ is a secondary or tertiary C₃₋₆ alkyl group. Examples of C₄₋₇ alkyl include n-, sec- and tert-butyl, n-pentyl, n-heptyl, and iso-butyl, 3-methylbutyl, and tert-butylmethyl.

Examples of R₅/R₆ when C₃₋₈ cycloalkyl-C₁₋₂ alkyl include in particular those wherein the cycloalkyl moiety is cyclohexyl or cyclopropyl. Examples include cyclopropylmethyl, cyclobutylmethyl, cyclopentylmethyl, cyclohexylmethyl, cyclopropylethyl, cyclobutylethyl, cyclopentylethyl, cyclohexylethyl, tert-butylmethyl, iso-propylmethyl, iso-propylethyl and tert-butylethyl.

R₅/R₆ may in particular be cyclopropylmethyl, cyclohexylmethyl, iso-propylmethyl, tert-butylmethyl or iso-propylethyl, preferably tert-butylmethyl.

Examples of R₅/R₆ when C₂₋₇ alkenyl-C₁₋₄ alkyl include prop-2-enyl, but-2-enyl, but-3-enyl, 1-methylenepropyl and 1-methyl-prop-2-enyl in their E and Z forms when stereoisomerism exists.

R₅/R₆ is preferably methyl or ethyl, most preferably methyl.

The pharmaceutically acceptable salts of the compounds of the formula (I) include acid addition salts with conventional acids such as hydrochloric, hydrobromic, boric, phosphoric, sulphuric acids and pharmaceutically acceptable organic acids such as acetic, tartaric, lactic, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (I) are usually acid addition salts with acids such as hydrochloric, hydrobromic, phosphoric, sulphuric, citric, tartaric, lactic and acetic acid.

Preferably the acid addition salt is the hydrochloride salt.

Examples of pharmaceutically acceptable salts include quaternary derivatives of the compounds of formula (I) such as the compounds quaternised by compounds R₁₀-T wherein R₁₀ is C₁₋₆ alkyl, phenyl-C₁₋₆ alkyl or C₅₋₇ cycloalkyl, and T is a radical corresponding to an anion of an acid. Suitable examples of R₁₀ include methyl, ethyl and n- and iso-propyl; and benzyl and phenethyl. Suitable examples of T include halide such as chloride, bromide and iodide.

The compounds of formula (I) may also form internal salts such as pharmaceutically acceptable N-oxides.

The compounds of the formula (I), their pharmaceutically acceptable salts, (including quaternary derivatives and N-oxides) may also form pharmaceutically acceptable solvates, such as hydrates, which are included wherever a compound of formula (I) or a salt thereof is herein referred to.

It will of course be realised that some of the compounds of the formula (I) have chiral or prochiral centres and thus are capable of existing in a number of stereoisomeric forms including enantiomers. The invention extends to each of these stereoisomeric forms (including enantiomers), and to mixtures thereof (including racemates). The different stereoisomeric forms may be separated one from the other by the usual methods.

It will also be realised that compounds of formula (I) may adopt an endo or exo configuration with respect to L. The endo configuration is preferred.

A group of compounds within formula (I) is of formula (II): 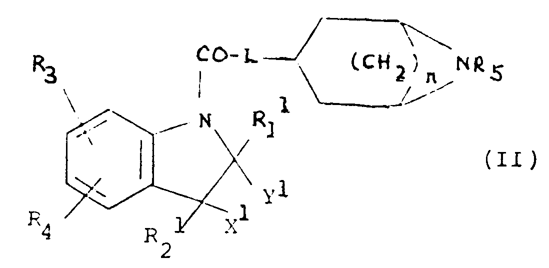
wherein X¹ and Y¹ are independently hydrogen, methyl or ethyl, or together are a bond, R₁¹ and R₂¹ are independently hydrogen, methyl or ethyl and the remaining variables are as defined in formula (I).

Examples of the variables and preferred variables are as so described for corresponding variables in relation to formula (I).

A further group of compounds within formula (I) is of formula (III): 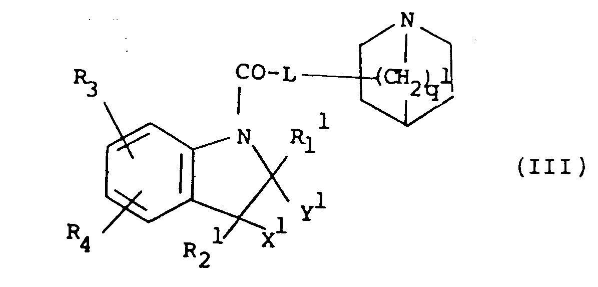
wherein q¹ is 1 or 2 and the remaining variables are as defined in formulae (I) and (II).

Examples of the variables and preferred variables are as so described for the corresponding variables in formula (I).

There is a further group of compounds within formula (I) of formula (IV): 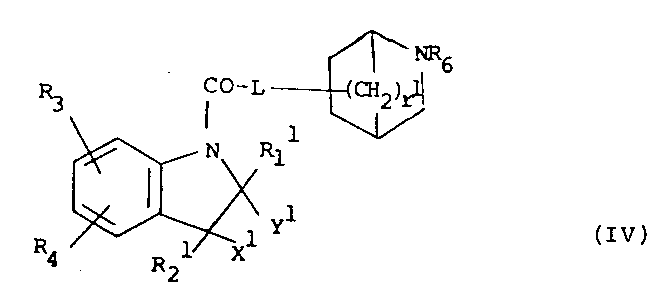
wherein r¹ is 1 or 2 and the remaining variables are as defined in formulae (I) and (II).

Examples of the variables and preferred variables are so described as the corresponding variables in formula (I).

The invention also provides a process for the preparation of a compound of formula (I), or a pharmaceutically acceptable salt thereof, which process comprises reacting a compound of formula (V): 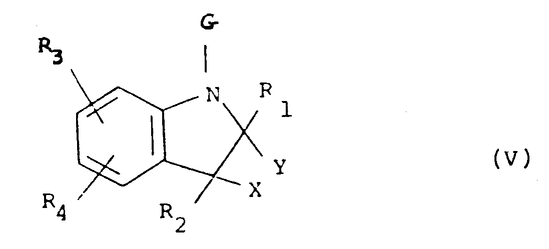
with a compound of formula (VI):

J-Z¹ (VI)

wherein
G is COQ₁, where Q₁ is a leaving group, or hydrogen; and, when G is COQ₁, J is NH₂, or OH or a reactive derivative thereof or, when G is hydrogen, J is a group containing an activated carbonyl group capable of forming a CO-L-linkage with the compound of formula (V); Z¹ is Z as defined or wherein R₅/R₆ is replaced by a hydrogenolysable protecting group; and the remaining variables are as hereinbefore defined; and thereafter optionally converting any R₃ and R₄ group to another R₃ and R₄ group respectively, converting Z¹, when other than Z, to Z; converting X and Y to other X and Y; and optionally forming a pharmaceutically acceptable salt of the resultant compound of formula (I).

Examples of leaving groups Q₁, displaceable by a nucleophile, include halogen such as chloro and bromo; C₁₋₄ alkoxy, such as CH₃O and C₂H₅O-; PhO-; activated hydrocarbyloxy, such as Cl₅C₆O- or Cl₃CO-; succinimidyloxy; and imidazolyloxy. Preferably Q₁ is halogen, most preferably chloro.

If a group Q₁ is a halide or imidazolyloxy, then the reaction is preferably carried out at non-extreme temperatures in an inert non-hydroxylic solvent, such as benzene, dichloromethane, toluene, diethyl ether, tetrahydrofuran (THF) or dimethylformamide (DMF). It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate. Temperatures of 0-100^{o}C, in particular 10-80^{o}C are suitable.

If a group Q₁ is C₁₋₄ alkoxy, phenoxy, activated hydrocarbyloxy or succinimidyloxy then the reaction is preferably carried out in an inert polar solvent, such as toluene or dimethylformamide. In this instance, it is preferred that the group Q₁ is Cl₃CO- or succinimidyloxy and that the reaction is carried out in toluene at reflux temperature.

When J is OH or a reactive derivative thereof, the reactive derivative is often a salt, such as the lithium, sodium or potassium salt.

When G is hydrogen, J-Z¹ may be a compound of formula (VII) or (VIII) when L is NH; or of formula (IX) when L is O:

O=C=N-Z¹ (VII)

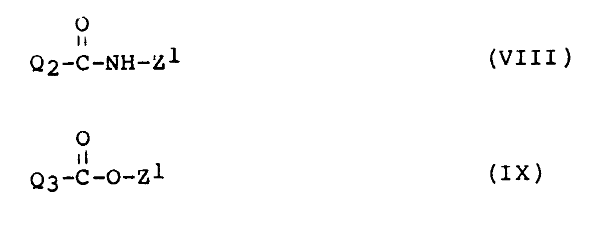

wherein
Z¹ is as hereinbefore defined, and Q₂ and Q₃ are leaving groups, preferably Cl₃CO and Cl respectively.

When J-Z¹ is of formula (VII), the reaction is preferably carried out in an inert solvent, under conventional conditions 0-100^{o}C.

Q₂ is a leaving group as defined for Q₁ hereinbefore; and the reaction is carried out in accordance with the conditions described herein for the reaction wherein G is COQ₁.

Examples of Q₃, displaceable by a nucleophile, include halogen, such as chloro and bromo; and activated hydrocarbyloxy, such as Cl₅C₆O- and Cl₃CO.

If a group Q₃ is a halide, the reaction is carried out as described above for Q₁ halide.

If Q₃ is activated hydrocarbyloxy, the reaction is carried out as described for Q₁ activated hydrocarbyloxy.

It will be apparent that compounds of the formula (I) containing an R₃ or R₄ group which is convertible to another R₃ or R₄ group are useful novel intermediates. A number of such conversions is possible not only for the end compounds of formula (I), but also for their intermediates as follows:
(i) a hydrogen substituent is convertible to a nitro substituent by nitration;
(ii) a nitro substituent is convertible to an amino substituent by reduction;
(iii) a C₁₋₇ acylamino substituent is convertible to an amino substituent by deacylation;
(iv) an amino substituent is convertible to a C₁₋₄ acylamino substituent by acylation with a carboxylic acid derivative;
(v) a hydrogen substituent is convertible to a halogen substituent by halogenation;
(vi) a C₁₋₆ alkylthio or C₁₋₆ alkylsulphinyl substituent is convertible to a C₁₋₆ alkylsulphinyl or a C₁₋₆ alkylsulphonyl substituent respectively by oxidation;
(vii) an amino, aminocarbonyl, aminosulphonyl, aminosulphonylamino or N-(aminosulphonyl)-N-C₁₋₄ alkylamino substituent is convertible to a corresponding substituent substituted by one or two groups selected from C₁₋₆ alkyl, C₃₋₈ cycloalkyl, C₁₋₄ alkyl or phenyl C₁₋₄ alkyl groups any of which phenyl groups may be substituted by one or more groups selected from halogen, trifluoromethyl, C₁₋₆ alkyl, C₁₋₆ alkoxy and nitro, or disubstituted by C₄₋₅ polymethylene, by N-alkylation;
(viii) an amino substituent is convertible to a C₁₋₆ alkylsulphonylamino group or an aminosulphonylamino group optionally N-substituted as defined by acylation with a C₁₋₆ alkylsulphonyl chloride or di-substituted aminosulphonyl chloride.
(ix) A C₁₋₄ alkylamino substituent group is convertible to a N-(C₁₋₆ alkylsulphonyl)N-C₁₋₄ alkylamino group or an N-(amino sulphonyl)N-C₁₋₄ alkylamino group optionally N-substituted as defined by acylation with a C₁₋₆ alkylsulphonyl chloride or di-substituted aminosulphonyl chloride.

Conversions (i) to (ix) are only exemplary and are not exhaustive of the possibilities.

In regard to (i), nitration is carried out in accordance with known procedures.

In regard to (ii), the reduction is carried out with a reagent suitable for reducing nitroanisole to aminoanisole.

In regard to (iii), deacylation is carried out by treatment with a base, such as an alkali metal hydroxide.

In regard to (iv), (viii), and (ix) the acylation is carried out with an acylating agent, such as the corresponding acid or acid chloride. Formylation is carried out with the free acid.

In regard to (v), halogenation is carried out with conventional halogenating agents.

In regard to (vi), oxidation is carried out at below ambient temperatures in a non-aqueous solvent, such as a chlorinated hydrocarbon, in the presence of an organic peracid, such as 3-chloroperbenzoic acid, or in water in the presence of a soluble strong inorganic oxidant, such as an alkali metal permanganate or in aqueous hydrogen peroxide. It will be realised that this process may also N-oxidise the N- moiety of a side chain (a), (b) or (c) and suitable precautions will routinely be taken by those skilled in the art.

In regard to (vii), alkylation is carried out with a corresponding alkylating agent such as the chloride or bromide under conventional conditions.

Z¹ when other than Z may have a hydrogenolysable protecting group which is benzyl optionally substituted by one or two groups as defined for R₃ and R₄. Such benzyl groups may, for example, be removed, when R₃ or R₄ is not halogen, by conventional transition metal catalysed hydrogenolysis to give compounds of the formula (X): 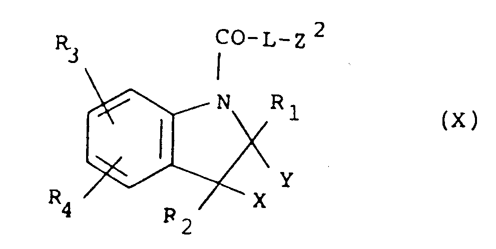
wherein Z² is of formula (d) or (e) 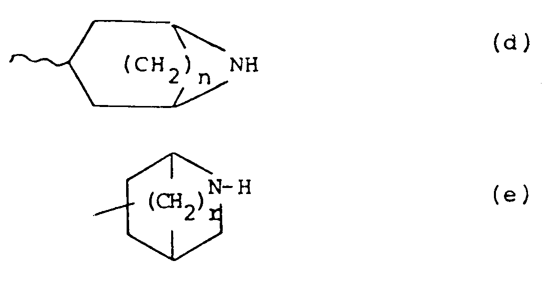
wherein the variables are as defined in formula (I).

This invention also provides a further process for the preparation of a compound of the formula (I) which comprises N-alkylating a compound of formula (X), and optionally forming a pharmaceutically acceptable salt, of the resulting compound of the formula (I).

In this further process of the invention 'N-alkylation' comprises the substitution of the N-atom depicted in formula (X) by any group R₅/R₆ as hereinbefore defined. This may be achieved by reaction of the compound of formula (X) with a compound R₅Q₄ or R₆Q₄ wherein R₅ and R₆ are as hereinbefore defined and Q₄ is a leaving group.

Suitable values for Q₄ include groups displaced by nucleophiles such as Cl, Br, I, OSO₂CH₃ or OSO₂C₆H₄pCH₃.

Favoured values for Q₄ include Cl, Br and I.

The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above.

Alternatively, 'N-alkylation' may be effected under conventional reductive alkylation conditions when the group R₅ or R₆ in the compound of formula (I) contains a methylene group adjacent to the N-atom in the bicycle.

Interconverting R₅ or R₆ in the compound of the formula (X) before coupling with the compound of the formula (V) is also possible. Such interconversions are effected conveniently under the above conditions. It is desirable to protect any amine function with a group readily removable by acidolysis such as a C₂₋₇ alkanoyl group, before R₅/R₆ interconversion.

When R₅ or R₆ in the compound of formula (VI) contains a methylene group adjacent to the N-atom in the bicycle it is often convenient in the preparation of such a compound of formula (VI) to prepare the corresponding compound wherein the methylene group is replaced by -CO-, or for R₅ or R₆ is methyl, where the methyl group is replaced by esterified carboxyl. Such compounds may then be reduced using a strong reductant such as lithium aluminium hydride to the corresponding compound of formula (V).

The compounds of formula (V) and (VI) are known or are preparable analogously to, or routinely from, known compounds. Intermediates of formula (V) wherein G is H and X and Y are hydrogen may be prepared from the corresponding intermediate wherein X and Y are a bond. Intermediates of formula (V) wherein G is COQ₁ excluding 1-(2,3-dihydro)indolyl carbonyl chloride form an aspect of the invention.

Compounds of the formula (VI) wherein Z is of formula (c) may be prepared as described in European Patent Publication No. 115933 or by analogous methods thereto.

Compounds of the formula (X) are novel and form an aspect of the invention.

It will be realised that in the compound of the formula (I) the -CO-L-linkage may have an endo or exo orientation with respect to the ring of the bicyclic moiety to which it is attached. A mixture of endo and exo isomers of the compound of the formula (I) may be synthesised non-stereospecifically and the desired isomer separated conventionally therefrom e.g. by chromatography; or alternatively the endo and exo isomer may if desired be synthesised from the corresponding endo or exo form of the compound of the formula (VI).

Compounds of the formula (I) wherein X and Y are both hydrogen may be converted to the corresponding compounds wherein X and Y are a bond by conventional oxidation, and this is the preferred method of preparation when X and Y are a bond. Compounds of the formula (I) wherein X and Y are a bond may be converted to the corresponding compounds wherein X and Y are hydrogen by reduction; however it is preferred that this is carried out on the compound of formula (V) wherein G is H prior to coupling.

Pharmaceutically acceptable salts of the compounds of this invention may be formed conventionally. The acid addition salts may be formed for example by reaction of the base compound of formula (I) with a pharmaceutically acceptable organic or inorganic acid.

The compounds of the present invention are 5-HT antagonists and it is thus believed may generally be used in the treatment or prophylaxis of migraine, cluster headaches and trigeminal neuralgia. Compounds which are 5-HT antagonists may also be of potential use in the treatment of CNS disorders such as anxiety and psychosis; arrhythmia, obesity and irritable bowel syndrome.

The compounds of the present invention also have anti-emetic activity; in particular that of preventing cytotoxic agent or radiation induced nausea and vomiting. Examples of cytotoxic agents include cisplatin, doxorubicin and cyclophosphamide.

The compounds of the present invention also have gastric motility enhancing activity, useful in the treatment of disorders such as retarded gastric emptying, dyspepsia, flatulence, oesophagal reflux and peptic ulcer.

The invention also provides a pharmaceutical composition comprising a compound of formula (I), or a pharmaceutically acceptable salt thereof, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides the use of a compound of formula (I) or 2,3-dihydroindole-1-carboxylic acid 1-azabicyclo[2.2.2]oct-3-yl ester, or a pharmaceutically acceptable salt thereof, in the preparation of a medicament for the treatment or prophylaxis of migraine, cluster headache, trigeminal neuralgia, emesis, CNS disorders such as anxiety and psychosis, arrhythmia, obesity and/or irritable bowel syndrome.

An amount effective to treat the disorders hereinbefore described depends on the relative efficacies of the compounds of the invention, the nature and severity of the disorder being treated and the weight of the mammal. However, a unit dose for a 70kg adult will normally contain 0.05 to 1000mg for example 0.1 to 500mg, of the compound of the invention. Unit doses may be administered once or more than once a day, for example, 2, 3 or 4 times a day, more usually 1 to 3 times a day, that is in the range of approximately 0.0001 to 50mg/kg/day, more usually 0.0002 to 25 mg/kg/day.

No adverse toxicological effects are indicated at any of the aforementioned dosage ranges.

The invention also provides a compound of formula (I) or a pharmaceutically acceptable salt thereof for use as an active therapeutic substance, in particular for use in the treatment of migraine, cluster headache, trigeminal neuralgia, emesis, anxiety, psychosis, arrhythmia, obesity and/or irritable bowel syndrome.

The following Examples illustrate the preparation of compounds of formula (I); the following descriptions illustrate the preparation of intermediates.

N.B. Nomenclature is based on Chemical Abstracts Index Guide 1977 published by the American Chemical Society.

### Description 1

### 1-(2,3-Dihydro)-indolyltrichloromethyl carbamate (D1)

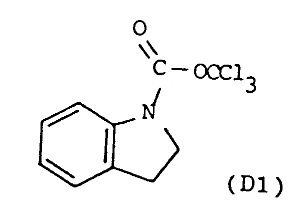

To 2,3-dihydroindole (5g) in dry dichloromethane (140ml) and triethylamine (5.85ml) at 0^{o} was added dropwise trichloromethyl chloroformate (5ml) in dry dichloromethane (20ml). The reaction mixture was stirred at room temperature for 2h, then washed with water (5ml) and 5N hydrochloric acid solution (5ml). The organic phase was dried (Na₂SO₄), the solvent evaporated in vacuo and the residue purified by filtration through a short alumina column, eluting with dichloromethane to give the title compound (D1) (8.5g, 72%) as a buff solid m.p. 59-60^{o}.
¹H-NMR (CDCl₃) 60MHz
- δ: 7.85-7.55 (m, 1H)
7.30-6.70 (m, 3H)
4.25-3.70 (m, 2H)
3.25-2.80 (m, 2H)

### Description 2

### 2,3-Dihydro-3-methylindole (D2)

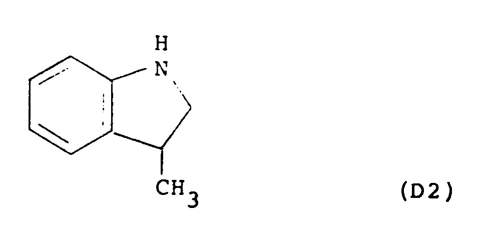

Following the procedure outlined by G.W. Gribble and J.H. Hoffman, Synthesis 859, 1977, 3-methyl indole (5g) was converted to the title compound (D2) (4.17g, 82%). ¹H NMR (CDCl₃) 60MHz

- δ: 7.30-6.30 (m, 4H)
3.80-2.80 (m, 4H)
1.30 (d, 3H)

### Description 3

### 2,3-Dihydro-5-fluoroindole (D3)

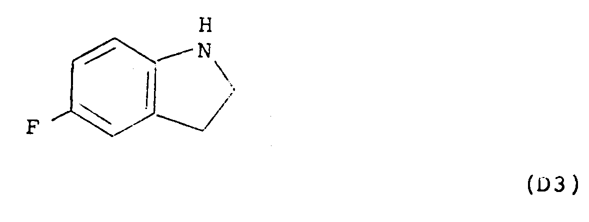

Following the procedure outlined in Description 2, 5-fluoroindole (3g) was converted to the title compound (D3) (2.54g, 84%).
¹H-NMR (CDCl₃) 60MHz
- δ: 7.05-6.10 (m, 3H)
4.10-2.60 (m, 5H)

### Description 4

### 2,3-Dihydro-5-chloroindole (D4)

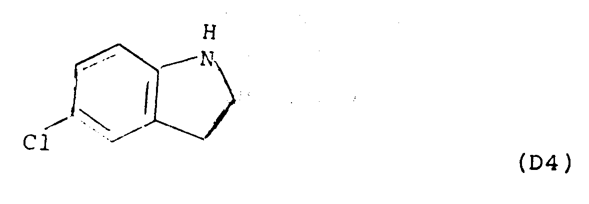

Following the procedure outlined in Description 2, 5-chloroindole (0.86g) was converted to the title compound (DA) (0.84g, 97%).
¹H-NMR (CDCl₃) 60MHz
- δ: 7.30-6.65 (m, 2H)
6.60-6.25 (m, 1H)
4.10-3.25 (m, 3H)
3.20-2.70 (m, 2H)

### Description 5

### 2,3-Dihydro-5-methoxyindole (D5)

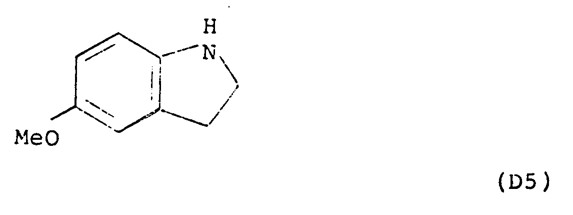

A solution of 5-methoxyindole (1g) in glacial acetic acid (20ml) was hydrogenated over platinum oxide (0.27g) at room temperature. After absorption of the theoretical amount of hydrogen (153ml), the catalyst was filtered off and the solvent evaporated in vacuo. The residue was basified with saturated potassium carbonate solution and extracted with diethyl ether. The organic phase was dried (Na₂SO₄), the solvent evaporated in vacuo to give the title compound (D5) (0.43g, 42%).
¹H-NMR (CDCl₃) 60MHz
- δ: 6.85-6.35 (m, 3H)
3.65 (s, 3H)
3.60-2.70 (m, 5H)

### Description 6

### 2,3-Dihydro-3-ethylindole (D6)

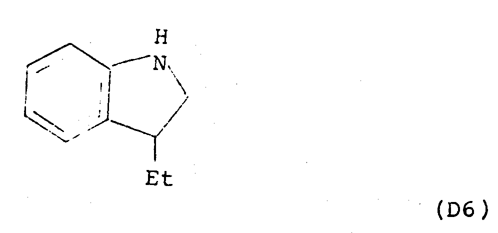

Following the procedure outlined in Description 2, 3-ethylindole (2.3g) (J.T. Fitzpatrick and R.D. Hiser, J. Org. Chem., 22, 1703-4, 1957) was converted to the title compound (D6) (1.3g, 56%).
¹H-NMR (CDCl₃) 60MHz
- δ: 7.20-6.40 (m, 4H)
3.90-2.90 (m, 4H)
2.10-0.8 (m, 2H)
0.9 (t, 3H)

### Description 7

### 1-(2,3-Dihydro-3-methyl)indolyl-O-(1-succinimidyl)carbamate (D7)

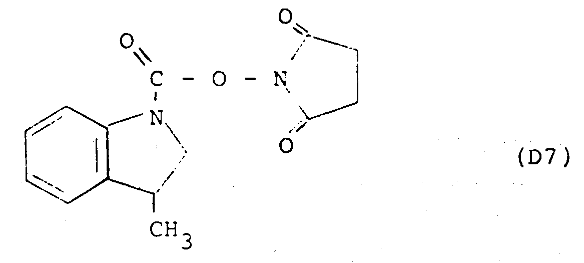

N,N-Disuccinimidyl carbonate (8.03g) and 2,3-dihydro-3-methylindole (D2) (4.17g) in dry toluene (150ml) was stirred at room temperature overnight. The solvent was evaporated in vacuo and the residue dissolved in dichloromethane, washed with 5N hydrochloric acid solution (10ml), saturated potassium bicarbonate (10ml) and brine (30ml). The organic phase was dried (Na₂SO₄), evaporated in vacuo and the residue purified by filtration through a short silica column, eluting with dichloromethane to give the title compound (D7) (6.85g, 80%).
¹H-NMR (CDCl₃) 60MHz
- δ: 7.85-6.80 (m, 4H)
4.60-4.00 (m, 1H)
3.95-3.10 (m, 2H)
2.75 (s, 4H)
1.30 (bd, 3H)

### Description 8

### 1-(2,3-Dihydro-5-fluoro)indolyl-O-(1-succinimidyl)carbamate (D8)

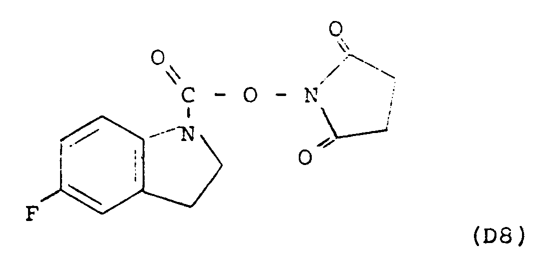

Following the procedure outlined in Description 7, reaction of N,N-disuccinimidyl carbonate (4.75g) with 2,3-dihydro-5-fluoroindole (D3) afforded the title compound (D8) (5g, 97%).
¹H-NMR (CDCl₃) 60MHz
- δ: 7.90-7.60 (m, 1H)
7.30-6.60 (m, 3H)
4.40-4.00 (m, 2H)
3.40-2.90 (m, 2H)
2.85 (s, 4H)

### Description 9

### 1-(2,3-Dihydro-5-methoxy)indolyl trichloromethyl carbamate (D9)

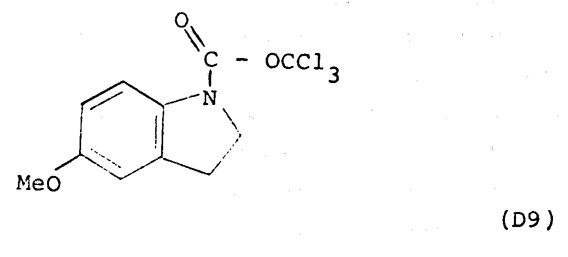

Following the procedure outlined in Description 1, reaction of 2,3-dihydro-5-methoxyindole (D5) (0.43g) with trichloromethylchloroformate (0.35ml) afforded the title compound (D9) (0.52g, 58%).
¹H-NMR (CDCl₃) 60MHz
- δ: 7.88-7.58 (m, 1H)
6.85-6.48 (m, 2H)
4.35-3.80 (m, 2H)
3.70 (s, 3H)
3.35-2.80 (m, 2H)

### Description 10

### 1-(2,3-Dihydro)indolylcarbonyl chloride (D10)

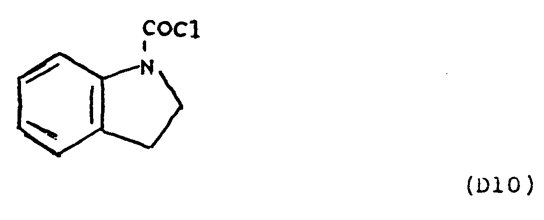

To phosgene [110ml (12.5% w/w solution in toluene)] in dry dichloromethane (150ml) at 0^{o} was added dropwise a solution of triethylamine (17ml) and freshly distilled 2,3-dihydroindole (14.5g) in dry dichloromethane (100ml). The reaction mixture was then stirred at 0^{o} for 1h, and then poured into pentane (2.51), washed with 5N sulphuric acid solution (100ml) and brine (100ml). The organic phase was dried (Na₂SO₄), the solvent evaporated in vacuo and the residue triturated with 60/80 pet. ether to give the title compound (D10) (18.37g, 83%).

### Description 11

### 1-(2,3-Dihydro-3-ethyl)indolylcarbonyl chloride (D11)

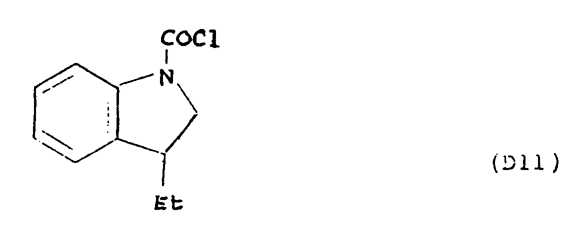

Following the procedure outlined in Description 10, reaction of 2,3-dihydro-3-ethylindole (D6) (1.25g) with phosgene [7.7ml (12.5% w/w solution in toluene)] afforded the title compound (D11) (1.6g, 90%).

### Description 12

### 1-(2,3-Dihydro-5-nitro)indolyl-trichloromethyl carbamate (D12)

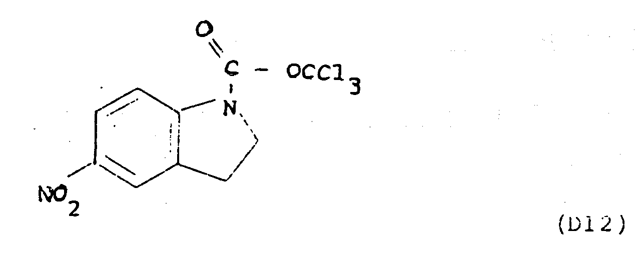

Following the procedure outlined in Description 1, reaction of 2,3-dihydro-5-nitroindole, (4.72g) with trichloromethylchloroformate (3.44ml) afforded the title compound (D12) (5.5g, 59%)
¹H-NMR (CDCl₃) 60MHz
- δ: 8.80-7.10 (m, 3H)
4.70-3.90 (m, 2H)
3.50-2.95 (m, 2H)

### Description 13

### 1-[1-(2,3-Dihydro-6-nitro)indolylcarbonyl]imidazole (D13)

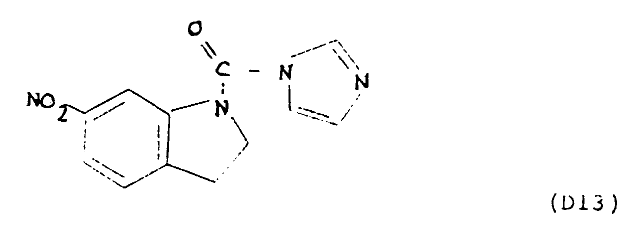

2,3-Dihydro-6-nitroindole (3g) and 1,1'-carbonyldiimidazole (2.96g) in dry toluene (75ml) was heated under reflux for 5h. The reaction mixture was cooled and the solvent evaporated in vacuo. The residue was dissolved in dichloromethane (100ml) and washed with 5N hydrochloric acid solution (10ml) and water (20ml). The organic phase was dried (Na₂SO₄) and the solvent evaporated in vacuo to give the title compound (D13) (4.7g, 100%).

### Description 14

### 1-(2,3-Dihydro-3,3-dimethyl)indolylcarbonyl chloride (D14)

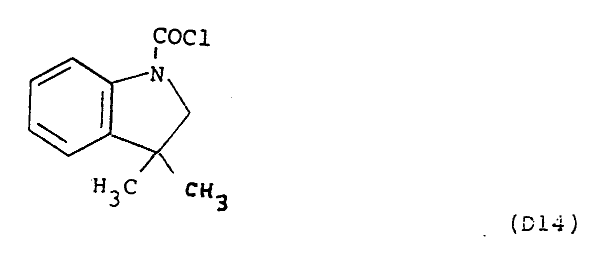

Following the procedure outlined in Description 10, reaction of 2,3-dihydro-3,3-dimethylindole (2.7g) with phosgene [16.5ml (12.5% w/w solution in toluene)] afforded the title compound (D14) (3.5g, 91%).

### Example 1

### endo-N-(9-Methyl-9-azabicyclo[3.3.1]non-3-yl)-2,3-dihydroindole-1-carboxamide (E1)

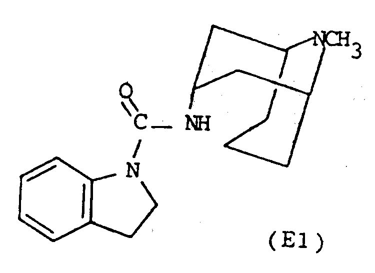

To 1-(2,3-dihydro)-indolyltrichloromethyl carbamate (D1) (3.64g) in dry toluene (100ml) was added endo-3-amino-9-methyl-9-azabicyclo[3.3.1]nonane (2g) in dry toluene (20ml). The reaction mixture was heated under reflux for 24h, then the solvent evaporated in vacuo. The residue was extracted with dichloromethane (200ml) and washed with saturated potassium carbonate solution (2 x 20ml). The organic phase was dried (Na₂SO₄) concentrated and the residue purified by column chromatography on alumina, eluting with CHCl₃ to give, after crystallisation from ethyl acetate, the title compound (E1) (2g, 52%) m.p. 176-8^{o}.
¹H-NMR (CDCl₃) 270MHz
- δ: 7.85 (d, 1H)
7.25-7.05 (m, 2H)
6.95-6.85 (m, 1H)
4.45-4.25 (m, 2H)
4.00-3.80 (t, 2H)
3.25-3.05 (m, 4H)
2.65-2.40 (m, 2H)
2.50 (s, 3H)
2.15-1.85 (m, 3H)
1.65-1.00 (m, 5H)

### Example 2

### endo-N-(8-Methyl-8-azabicyclo[3.2.1]oct-3-yl)-2,3-dihydroindole-1-carboxamide (E2)

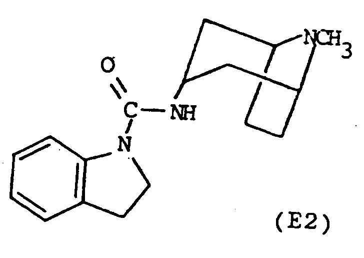

Following the procedure outlined in Example 1, reaction of 1-(2,3-dihydro)-indolyltrichloromethyl carbamate (D1) (0.64g) with endo-3-amino-8-methyl-8-azabicyclo[3.2.1]octane (0.32g) afforded the title compound (E2) m.p. 153-4^{o}
¹H-NMR (CDCl₃) 270MHz,
- δ: 7.85 (d, 1H)
7.25-7.10 (m, 2H)
6.95-6.85 (m, 1H)
4.95 (bd, 1H)
4.10 (q, 1H)
3.90 (t, 2H)
3.25-3.10 (m, 4H)
2.25-2.05 (m, 4H)
2.30 (s, 3H)
1.90-1.75 (m, 4H)

### Example 3

### endo -2,3-Dihydroindole-1-carboxylic acid 8-methyl-8-azabicyclo[3,2,1]oct-3-yl ester (E3)

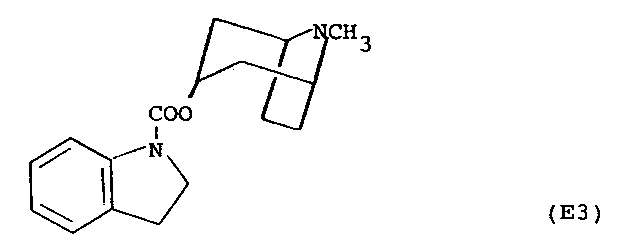

To 3-tropanol (1.13g) in diglyme (50ml) was added portionwise potassium t-butoxide (0.94g). The reaction mixture was stirred under an atmosphere of N₂ at room temperature for 1h and then the solvent was evaporated in vacuo. The resultant gum was redissolved in diglyme (50ml) and 1-(2,3-dihydro)indole trichloromethyl carbamate (D1) (1.5g) was added. The reaction mixture was heated under reflux for 36h, then cooled and evaporated in vacuo. The residue was dissolved in 5N hydrochloric acid solution (10ml) and washed with diethyl ether (30ml). The aqueous phase was basified with potassium carbonate and extracted with dichloromethane (3 x 75ml). The organic phase was dried (Na₂SO₄), the solvent evaporated in vacuo and the residue purified by column chromatography on alumina eluting with dichloromethane to give, after crystallisation from diethyl ether the title compound (E3) (0.5g, 31%). m.p. 133-4^{o}.
¹H-NMR (CDCl₃) 270MHz
- δ: 7.85 (bd, 1H)
7.22-7.12 (m, 2H)
7.00-6.92 (m, 1H)
5.05 (t, 1H)
4.06 (t, 2H)
3.28-3.08 (m, 4H)
2.32 (s, 3H)
2.32-1.75 (m, 8H)

### Example 4

### endo-N-(8-Methyl-8-azabicyclo[3.2.1]oct-3-yl)-2,3-dihydro-3-methylindole-1-carboxamide hydrochloride (E4)

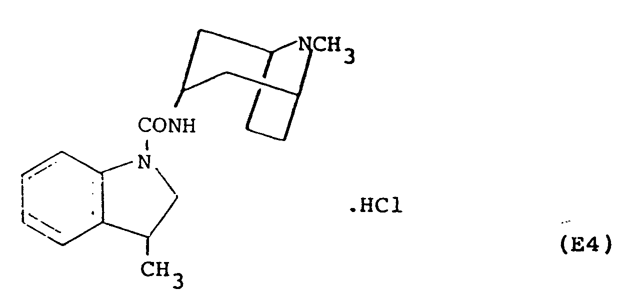

Triethylamine (1.8ml), 1-(2,3-dihydro-3-methyl)indolyl-O-(1-succinimidyl)carbamate (D7) (3.5g) and endo-3-amino-8-methyl-8-azabicyclo[3,2,1]octane (1.8g) were dissolved in dry toluene (100ml) and heated under reflux overnight. The reaction mixture was cooled and the solvent evaporated in vacuo. The residue was extracted with dichloromethane (200ml) and washed with saturated potassium carbonate solution (2 x 20ml). The organic phase was dried (Na₂SO₄), concentrated and the residue purified by column chromatography on alumina, eluting with chloroform. The product was isolated as the hydrochloride salt (E4) (0.97g, 23%). m.p. 268-70^{o}.
¹H-NMR (d₆-DMSO) 270MHz
10.35-10.05 (m, 1H)
7.75 (d, 1H)
7.25-7.05 (m, 2H)
6.95-6.85 (m, 1H)
6.29 (bs, 1H)
4.15 (t, 1H)
3.90-3.70 (m, 3H)
3.65-3.30 (m, 2H)
2.65 (s, 3H)
2.50-2.10 (m, 8H)
1.26 (d, 3H)

### Example 5

### endo-N-(8-Methyl-8-azabicyclo[3.2.1]oct-3-yl)-2,3-dihydro-3,3-dimethylindole-1-carboxamide (E5)

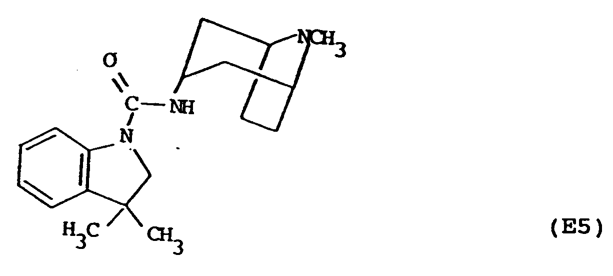

Following the procedure outlined in Example 14, reaction of 1-(2,3-dihydro-3,3-dimethyl)indolylcarbonyl chloride (D14) (1.2g) with endo-3-amino-8-methyl-8-azabicyclo[3.2.1]octane (0.8g) afforded the title compound (E5) (0.88g, 50%) m.p. 158-9°.
¹H-NMR CDCl₃
7.80 (d, 1H)
7.25-7.05 (m, 2H)
7.00-6.90 (m, 1H)
6.92 (bd, 1H)
4.08 (g, 1H)
3.60 (s, 2H)
3.30-3.15 (m, 2H)
2.35 (s, 3H)
2.40-2.10 (m, 4H)
1.95-1.65 (m, 4H)
1.35 (s, 6H)

### Example 6

### endo-N-(8-Methyl-8-azabicyclo[3,2,1]oct-3-yl)-3-methylindole-1-carboxamide hydrochloride (E6)

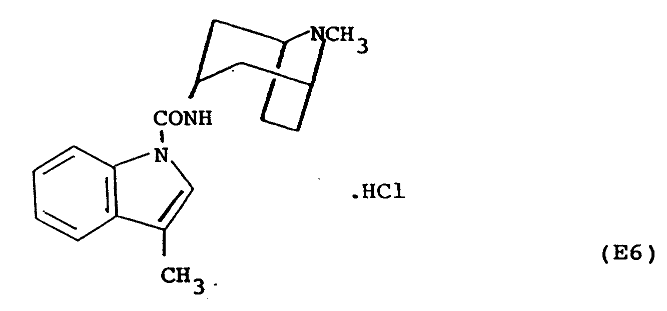

endo-N-(8-Methyl-8-azabicyclo[3.2.1]oct-3-yl)-2,3-dihyd ro-3-methylindole-1-carboxamide hydrochloride (E4) (0.5g) and 2,3-dichloro-5,6-dicyano-1,4-benzoquinone (0.41g) in dry chloroform (100ml) were heated under reflux for 6h. The reaction mixture was cooled and washed with saturated potassium carbonate solution (20ml). The organic phase was dried (Na₂SO₄), concentrated and the residue filtered through a short alumina column, eluting with chloroform. The product was isolated as the hydrochloride salt (E6) (0.2g, 40%). m.p. 158-61^{o}
¹H-NMR (d₆-DMSO) 400MHz
- δ: 10.50 (bs, 1H)
8.15 (d, 1H)
7.85 (bs, 1H)
7.65 (s, 1H)
7.55 (d, 1H)
7.30-7.15 (m, 2H)
4.00-3.75 (m, 3H)
2.65 (bs, 3H)
2.50-2.05 (m, 11H)

### Example 7

### endo-N-(8-Ethyl-8-azabicyclo[3.2.1]oct-3-yl)-2,3-dihydroindole-1-carboxamide (E7)

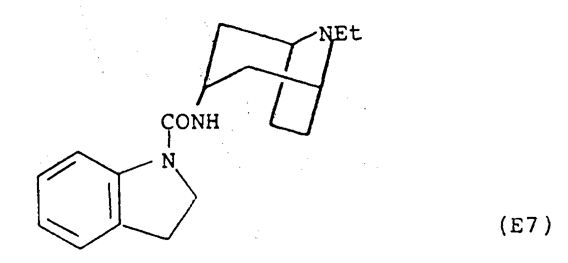

Following the procedure outlined in Example 1, reaction of 1-(2,3-dihydro)indole-trichloromethyl carbamate (D1) (0.91g) with endo-3-amino-8-ethyl-8-azabicyclo[3.2.1]octane (0.5g) afforded the title compound (E7) (0.24g, 25%) m.p. 140-1^{o.}
1H-NMR (CDCl₃) 270MHz
- δ: 7.85 (d, 1H)
7.25-7.10 (m, 2H)
6.95-6.85 (m, 1H)
4.95 (bd, 1H)
4.10 (q, 1H)
3.90 (t, 2H)
3.35 (bs, 2H)
3.15 (t, 2H)
2.45 (q, 2H)
2.38-2.20 (m, 2H)
2.18-2.00 (m, 2H)
1.95-1.65 (m, 4H)
1.10 (t, 3H)

### Example 8

### endo-N-(8-Methyl-8-azabicyclo[3.2.1]oct-3-yl)-5-fluoro-2,3-dihydroindole-1-carboxamide hydrochloride (E8)

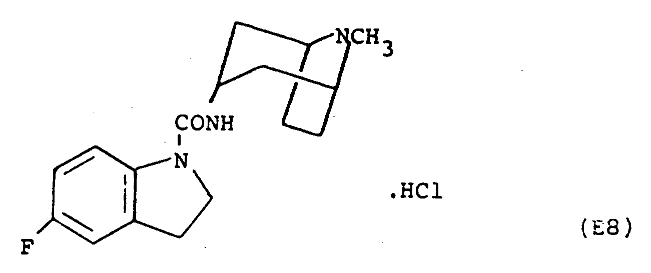

Following the procedure outlined in Example 4, reaction of 1-(2,3-dihydro-5-fluoro)indolyl-O-(1-succinimidyl)carbamate (D8) (3.59) with triethylamine (1.75ml) and endo-3-amino-8-methyl-8-azabicyclo[3.2.1]octane (1.76g) afforded the free base, which was converted to the hydrochloride salt (E8) (1.11g, 18%) m.p. 299-300^{o} (decomposition).
¹H-NMR (d₆-DMSO) 270MHz
- δ: 10.35-10.15 (m, 1H)
7.80-7.70 (m, 1H)
7.10-6.85 (m, 2H)
6.30 (bs, 1H)
4.05 (t, 2H)
3.90-3.70 (m, 3H)
3.10 (t, 2H)
2.65 (bs, 3H)
2.50-2.05 (m, 8H)

### Example 9

### endo-N-(8-Methyl-8-azabicyclo[3.2.1]oct-3-yl)-2,3-dihydro-5-chloroindole-1-carboxamide (E9)

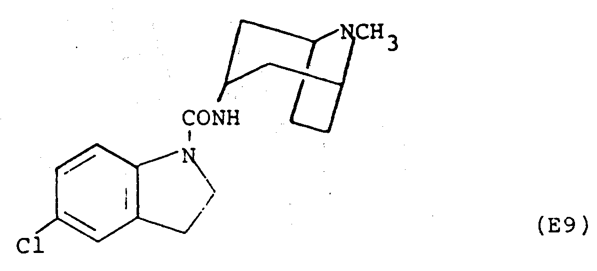

To phosgene [3.8ml (12.5% w/w solution in toluene)] in dry dichloromethane (50ml) was added dropwise 2,3-dihydro-5-chloroindole (D4) (0.83g) in CH₂Cl₂ (20ml). Triethylamine (0.83ml) was then added and the whole stirred at room temperature for 10 min. endo-3-Amino-8-methyl-8-azabicyclo[3.2.1]octane (0.83g) in dry dichloromethane (10ml) was added and the reaction mixture stirred at room temperature for 2h, then washed with saturated potassium bicarbonate solution (15ml) and brine (20ml). The organic phase was dried (Na₂SO₄), the solvent evaporated in vacuo and the residue column chromatographed on alumina eluting with chloroform to give, after crystallisation from ethyl acetate, the title compound (E9) (0.36g, 19%) m.p. 149-50^{o}.
¹H-NMR (CDCl₃) 400MHz
- δ: 7.81 (d, 1H)
7.15-7.05 (m, 2H)
4.90 (bd, 1H)
4.08 (q, 1H)
3.91 (t, 2H)
3.28-3.10 (m, 4H)
2.34 (s, 3H)
2.35-2.08 (m, 4H)
1.90-1.65 (m, 4H)

### Example 10

### endo-N-(8-Methyl-8-azabicyclo[3.2.1]oct-3-yl)2,3-dihydro-5-methoxyindole-1-carboxamide (E10)

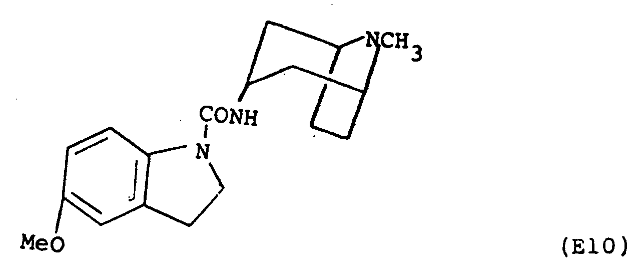

Following the procedure outlined in Example 1, reaction of 1-(2,3-dihydro-5-methoxy)indolyl trichloromethyl carbamate (D9) (0.48g) with endo-3-amino-8-methyl-8-azabicyclo[3.2.1]octane (0.23g) afforded the title compound (E10) (0.22g, 45%) m.p. 142-5^{o}.
¹H-NMR (CDCl₃) 270MHz
- δ: 7.75 (d, 1H)
6.80-6.65 (m, 2H)
4.88 (bd, 1H)
4.08 (q, 1H)
3.90 (t, 2H)
3.78 (s, 3H)
3.28-3.10 (m, 4H)
2.32 (s, 3H)
2.40-2.10 (m, 4H)
1.90-1.65 (m, 4H)

### Example 11

### endo-N-(8-Methyl-8-azabicyclo[3.2.1]oct-3-yl)indole-1-carboxamide hydrochloride (E11)

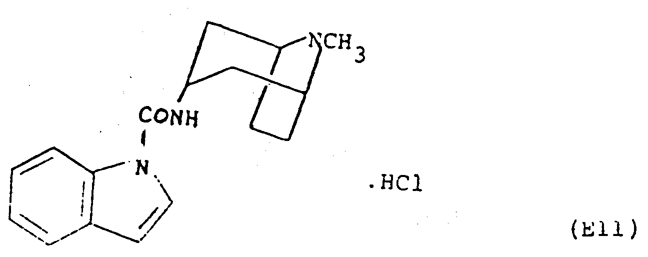

Following the procedure outlined in Example 6, reaction of endo-N-(8-methyl-8-azabicyclo[3.2.1]oct-3-yl)2,3-dihydroindole-1-carboxamide hydrochloride (E2) (0.46g) and 2,3-dichloro-5,6-dicyano-1,4-benzoquinone (0.44g) afforded the title compound (E11) (0.31g, 68%) m.p. 258-60^{o} (decomposition).
¹H-NMR (d₆-DMSO) 270MHz
- δ: 10.6-10.3 (m, 1H)
8.15-7.95 (m, 2H)
7.85 (d, 1H)
7.65-7.55 (m, 1H)
7.35-7.10 (m, 2H)
6.75-6.65 (m, 1H)
4.05-3.65 (m, 3H)
2.65 (bs, 3H)
2.60-2.00 (m, 8H)

### Example 12

### N-(1-Azabicyclo[2.2.2]oct-3-yl)2,3-dihydroindole-1-carboxamide hydrochloride (E12)

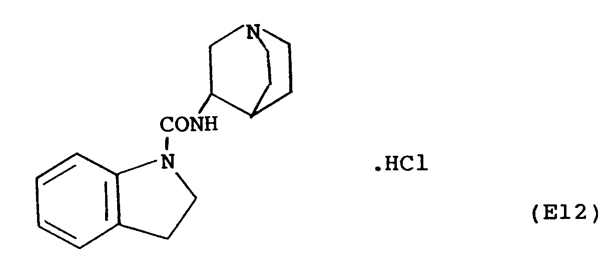

A mixture of 3-amino-1-azabicyclo[2.2.2]octane (0.5g) and triethylamine (0.7ml) in dry dimethylformamide (30ml) was heated at 50^{o} for 1h. The solution was cooled and added dropwise to a solution of 1-(2,3-dihydro)indolylcarbonyl chloride (D10) (0.46g) and triethylamine (0.35ml) in dry dimethylformamide (50ml) at 0^{o}. The reaction mixture was stirred at room temperature for 2h, the solvent was then evaporated in vacuo. The residue was dissolved in dichloromethane and washed with 10% sodium hydroxide solution (10ml). The organic phase was dried (Na₂SO₄), the solvent evaporated in vacuo and the residue was column chromatographed on alumina, eluting with chloroform. The product was isolated as the hydrochloride salt (E12) (0.16g, 21%) m.p. 138-40^{o}.
¹H-NMR (d₆-DMSO) 400MHz
- δ: 10.7-10.3 (m, 1H)
7.82 (d, 1H)
7.16 (d, 1H)
7.08 (t, 1H)
6.86 (t, 1H)
6.80 (d, 1H)
4.18-4.08 (m, 1H)
4.06-3.92 (m, 2H)
2.54 (t, 1H)
2.46-3.04 (m, 7H)
2.18-2.06 (m, 2H)
1.96-1.78 (m, 2H)
1.76-1.60 (m, 1H)

### Example 13

### 2,3-Dihydroindole-1-carboxylic acid 1-azabicyclo[2.2.2]oct-3-yl ester (E13)

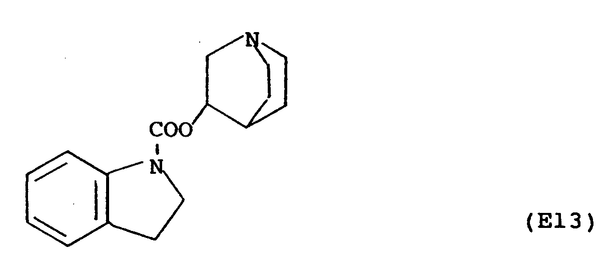

To 1-azabicyclo[2.2.2]octan-3-ol (1g) in dry tetrahydrofuran (75ml) at -78^{o} under an atmosphere of nitrogen, was added dropwise n-butyl lithium [5.2ml (1.6M solution in hexanes)]. The mixture was allowed to warm to room temperature and then stirred for 10 min. The reaction mixture was cooled to -78^{o} and 1-(2,3-dihydro)indolylcarbonyl chloride (1.43g) in dry tetrahydrofuran (20ml) was added dropwise. The reaction mixture was again allowed to warm to room temperature and stirred overnight. Water was added and the whole evaporated in vacuo, the residue was dissolved in dichloromethane (150ml) and washed with saturated potassium carbonate solution (30ml). The organic phase was dried (Na₂SO₄), the solvent was evaporated in vacuo and the residue column chromatographed on alumina, eluting with chloroform to give, after crystallisation from ethyl acetate, the title compound (E13) (0.29g, 14%) m.p. 124-5^{o}.
¹H-NMR (CDCl₃) 270MHz
- δ: 8.00-7.70 (m, 1H)
7.30-7.10 (m, 2H)
7.05-6.90 (m, 1H)
5.05-4.80 (m, 1H)
4.20-3.95 (m, 2H)
3.45-3.25 (m, 1H)
3.25-2.50 (m, 7H)
2.30-2.05 (m, 1H)
2.05-1.20 (m, 4H)

### Example 14

### endo-N-(8-Methyl-8-azabicyclo[3.2.1]oct-3-yl)-2,3-dihydro-3-ethylindole-1-carboxamide hydrochloride (E14)

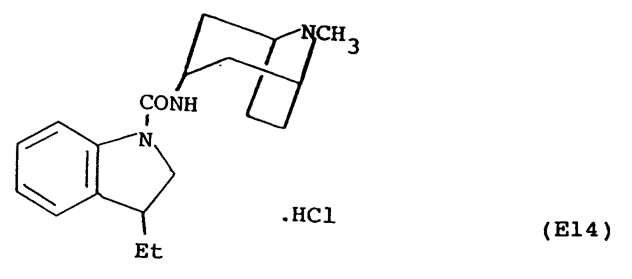

To 1-(2,3-dihydro-3-ethyl)indolylcarbonyl chloride (D11) (1g) in dry dichloromethane (100ml) was added dropwise a mixture of endo-3-amino-8-methyl-8-azabicyclo[3.2.1]octane (0.7g) and triethylamine (0.7ml) in dry dichloromethane (50ml). The reaction mixture was stirred at room temperature overnight, the solvent was then evaporated in vacuo. The residue dissolved in 5N hydrochloric acid solution (20ml) and washed with diethyl ether (50ml). The aqueous phase was basified with potassium carbonate and then extracted with dichloromethane (3 x 75ml). The organic phase was dried (Na₂SO₄), the solvent was evaporated in vacuo and the residue filtered through a short alumina column. The product was isolated as the hydrochloride salt (E14) (1.27g, 76%) m.p. 263-4^{o}.
¹H-NMR (d₆-DMSO) 270MHz
- δ: 10.80-10.20 (m, 1H)
7.80 (d, 1H)
7.25-7.05 (m, 2H)
6.95-6.80 (m, 1H)
6.32 (bs, 1H)
4.10 (t, 1H)
3.90-3.65 (m, 4H)
3.55-3.10 (m, 1H)
2.65 (bs, 3H)
2.60-2.00 (m, 8H)
1.90-1.65 (m, 1H)
1.60-1.40 (m, 1H)
0.92 (t, 3H)

### Example 15

### endo-N-(8-Methyl-8-azabicyclo[3.2.1]oct-3-yl)-3-ethylindole-1-carboxamide hydrochloride (E15)

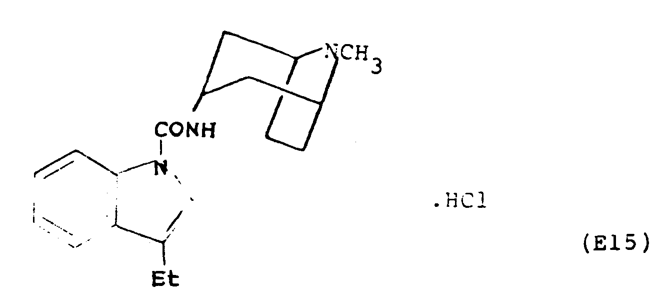

Following the procedure outlined in Example 6, reaction of endo-N-(8-methyl-8-azabicyclo[3.2.1]oct-3-yl)2,3-dihydro-3-ethylindole-1-carboxamide hydrochloride (E14) (1.01g) with 2,3-dichloro-5,6-dicyano-1,4-benzoquinone (0.8g) afforded the title compound (E15) (0.4g, 40%) m.p. 210-13^{o}.
¹H-NMR (d₆-DMSO) 270MHz
- δ: 10.90-10.50 (m, 1H)
8.15 (d, 1H)
7.90 (bs, 1H)
7.68 (s, 1H)
7.55 (d, 1H)
7.35-7.10 (m, 2H)
4.10-3.65 (m, 3H)
2.90-2.05 (m, 13H)
1.30 (t, 3H)

### Example 16

### endo-N-(8-Methyl-8-azabicyclo[3.2.1]oct-3-yl)-2,3-dihydro-5-nitroindole-1-carboxamide (E16)

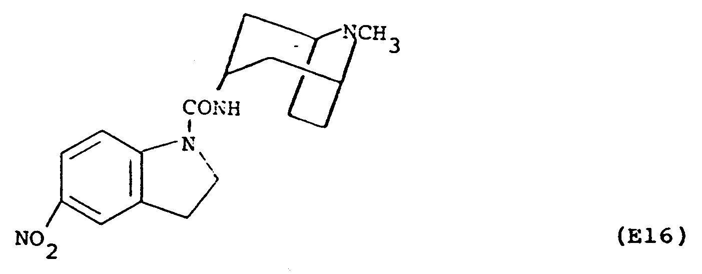

Following the procedure outlined in Example 1, reaction of 1-(2,3-dihydro-5-nitro)indolyl trichloromethyl carbamate (D12) (2g) with endo-3-amino-8-methyl-8-azabicyclo[3.2.1]octane (0.9g) afforded the title compound (E16) (1.25g, 62%) m.p. 176-8°.
¹H-NMR (CDCl₃) 270MHz
- δ: 8.18-7.95 (m, 3H)
5.05 (bd, 1H)
4.15-3.95 (m, 3H)
3.35-3.15 (m, 4H)
2.30 (s, 3H)
2.35-2.10 (m, 4H)
1.85-1.60 (m, 4H)

### Example 17

### endo-N-(8-Methyl-8-azabicyclo[3.2.1]oct-3-yl)-2,3-dihydro-6-nitroindole-1-carboxamide hydrochloride (E17)

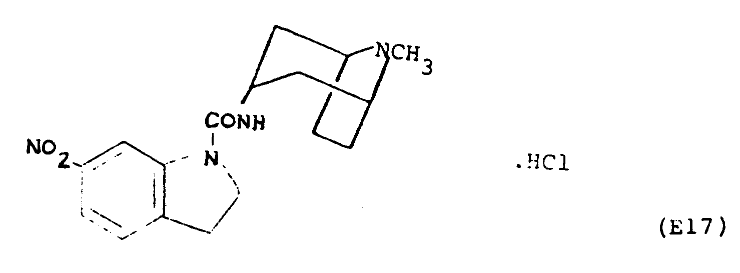

Following the procedure outlined in Example 1, reaction of 1-[1-(2,3-dihydro-6-nitro)indolylcarbonyl]imidazole (D13) (4.7g) with endo-3-amino-8-methyl-8-azabicyclo[3.2.1]octane (2.55g) afforded the title compound (E17) m.p. 245-7^{o} (decomposition).
¹H-NMR (d₆-DMSO) 270MHz)
- δ: 10.15-9.95 (m, 1H)
8.55 (d, 1H)
7.85-7.70 (m, 1H)
7.45-7.35 (m, 1H)
6.65-6.55 (m, 1H)
4.15 (t, 2H)
3.90-3.70 (m, 1H)
3.60-3.35 (m, 2H)
3.30-3.15 (t, 2H)
2.65 (d, 3H)
2.45-2.00 (m, 8H)

### Example 18

### N-(1-Azabicyclo[2.2.2]oct-3-yl)2,3-dihydro-3,3-dimethyl indole-1-carboxamide hydrochloride (E18)

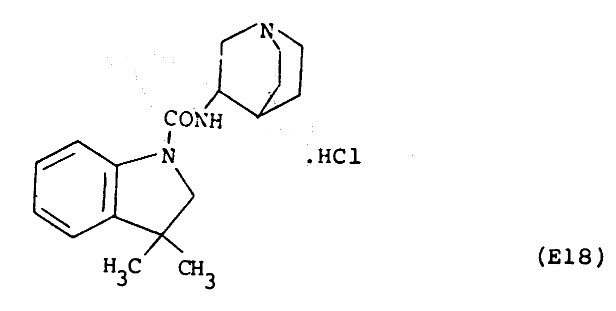

To a solution of 3-amino-1-azabicyclo[2.2.2]octane dihydrochloride (0.87g) in water (1.5ml) was added dry dimethylformamide (30ml) and triethylamine (2ml). The mixture was stirred at room temperature for 5 min, then a solution of 1-(2,3-dihydro-3,3-dimethyl)indolylcarbonyl chloride (D14) in dry dimethylformamide (20ml) was added dropwise. The reaction mixture was stirred at room temperature for 18h, the solvent was then evaporated in vacuo. The residue was dissolved in 5N hydrochloric acid solution (25ml) and washed with diethyl ether (50ml). The aqueous phase was basified with potassium carbonate and then extracted with dichloromethane (3 x 75ml). The organic phase was dried (Na₂SO₄), the solvent was evaporated in vacuo and the residue crystallised from ethyl acetate/diethyl ether to give the title compound (E18) m.p. 174-6^{o}.
¹H-NMR (CDCl₃) 270MHz
11.40 (bs, 1H)
7.95 (d, 1H)
7.25-7.05 (m, 2H)
6.95 (t, 1H)
6.65 (bd, 1H)
4.60-4.40 (m, 1H)
4.28 (dd, 1H)
4.10-3.80 (m, 3H)
3.55-3.35 (m, 1H)
3.30-3.00 (m, 3H)
2.50-2.30 (m, 2H)
2.25-1.90 (m, 1H)
1.95-1.60 (m, 2H)
1.35 (s, 6H)

### Pharmacology

### Antagonism of the von Bezold-Jarisch reflex

The compounds were evaluated for antagonism of the von Bezold-Jarisch reflex evoked by 5-HT in the anaesthetised rat according to the following method:

Male rats, 250-350g, were anaesthetised with urethane (1.25g/kg intraperitoneally) and blood pressure and heart rate recorded as described by Fozard J.R. et al., J. Cardiovasc. Pharmacol. 2, 229-245 (1980). A submaximal dose of 5-HT (usually 6µg/kg) was given repeatedly by the intravenous route and changes in heart rate quantified. Compounds were given intravenously and the concentration required to reduce the 5HT-evoked response to 50% of the control response (ED₅₀) was then determined.

The results were as shown in Table 1.

**Table 1**

| Compound of Example No. | ED₅₀ µg/kg i.v |
|---|---|
| 1 | 12.5 |
| 2 | 1.4 |
| 3 | 5 |
| 4 | 3 |
| 5 | 0.58 |
| 6 | 1.6 |
| 7 | >10 |
| 8 | 17 |
| 9 | >10 |
| 10 | 7 |
| 11 | 7.7 |
| 12 | 4.4 |
| 13 | 3.9 |
| 14 | 1.0 |
| 15 | 2.0 |
| 16 | >10 |
| 17 | >10 |
| 18 | 5.3 |

## Claims (Claims for the following Contracting State(s): BE, CH, DE, FR, GB, GR, IT, LI, LU, NL, SE)

1. A compound of formula (I), or a pharmaceutically acceptable salt thereof: 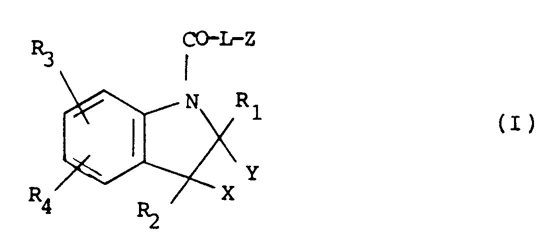 wherein
L is NH or O;
X and Y are independently selected from hydrogen or C₁₋₄ alkyl, or together are a bond;
R₁ and R₂ are independently selected from hydrogen, C₁₋₆ alkyl, C₂₋₆ alkenyl-C₁₋₄ alkyl, or together are C₂₋₄ polymethylene;
R₃ and R₄ are independently selected from hydrogen, halogen, CF₃, C₁₋₆ alkyl, C₁₋₆ alkoxy, C₁₋₆ alkylthio, C₁₋₇ acyl, C₁₋₇ acylamino, C₁₋₆ alkylsulphonylamino, N-(C₁₋₆ alkylsulphonyl)-N-C₁₋₄ alkylamino, C₁₋₆ alkylsulphinyl, hydroxy, nitro or amino, aminocarbonyl, aminosulphonyl, aminosulphonylamino or N-(aminosulphonyl)-C₁₋₄ alkylamino optionally N-substituted by one or two groups selected from C₁₋₆ alkyl, C₃₋₈ cycloalkyl, C₃₋₈ cycloalkyl C₁₋₄ alkyl, phenyl or phenyl C₁₋₄ alkyl groups or optionally N-disubstituted by C₄₋₅ polymethylene;
Z is a group of formula (a), (b) or (c) 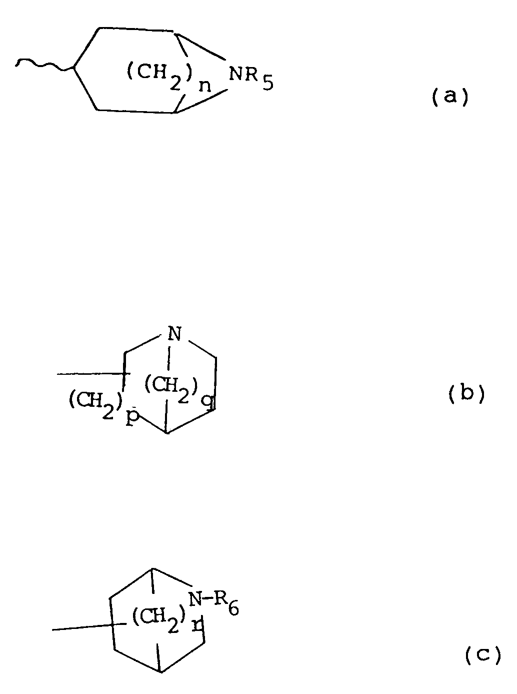 wherein n is 2 or 3; p is 1 or 2; q is 1 to 3; r is 1 to 3; and
R₅ or R₆ is C₁₋₇ alkyl, C₃₋₈ cycloalkyl, C₃₋₈ cycloalkyl-C₁₋₂ alkyl or C₂₋₇ alkenyl-C₁₋₄ alkyl; with the proviso that the compound of formula (I) is other than 2,3-dihydroindole-1-carboxylic acid 1-azabicyclic [2.2.2]oct-3-yl ester.

2. A compound according to claim 1 of formula (II): 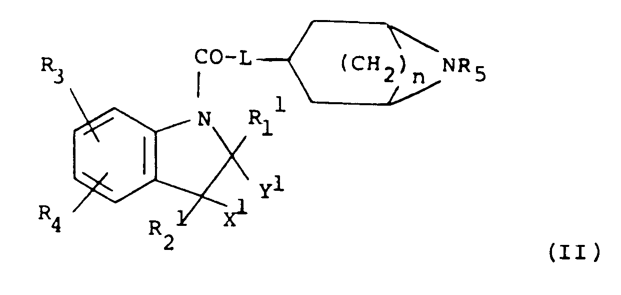 wherein X¹ and Y¹ are independently hydrogen, methyl or ethyl, or together are a bond, R₁¹ and R₂¹ are independently hydrogen, methyl or ethyl and the remaining variables are as defined in claim 1.

3. A compound according to claim 2 wherein n is 2.

4. A compound according to claim 2 or 3 wherein R₅ is methyl.

5. A compound according to claim 1 of formula (III): 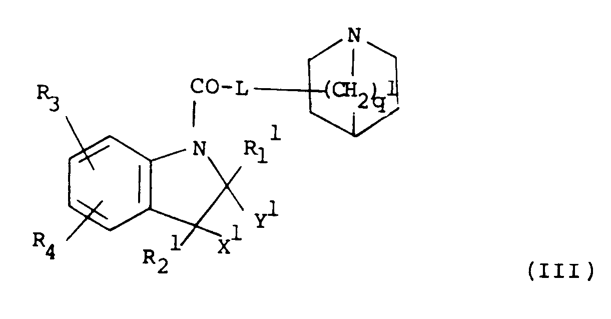 wherein q¹ is 1 or 2 and the remaining variables are as defined in claims 1 and 2.

6. A compound according to claim 5 wherein q¹ is 2.

7. A compound according to any one of claims 1 to 6 wherein one of R₃ and R₄ is hydrogen and the other is selected from hydrogen, 5-chloro and 5-fluoro.

8. A compound according to any one of claims 1 to 7 wherein L is NH.

9. endo-N-(9-Methyl-9-azabicyclo[3.3.1]non-3-yl) -2,3-dihydroindole-1-carboxamide,
endo-N-(8-methyl-8-azabicyclo[3.2.1]oct-3-yl) -2,3-dihydroindole-1-carboxamide,
endo(8-methyl-8-azabicyclo[3,2,1]oct-3-yl)-2,3-dihydroindole carboxylic acid ester,
endo-N-(8-methyl-8-azabicyclo[3.2.1]oct-3-yl) -2,3-dihydro-3-methylindole-1-carboxamide,
endo-N-(8-methyl-8-azabicyclo[3,2,1]oct-3-yl) -3-methyl-indole-1-carboxamide,
endo-N-(8-ethyl-8-azabicyclo[3.2.1]oct-3-yl) -2,3-dihydroindole-1-carboxamide,
endo-N-(8-methyl-8-azabicyclo[3.2.1]oct-3-yl) -5-fluoro-2,3-dihydroindole-1-carboxamide,
endo-N-(8-methyl-8-azabicyclo[3.2.1]oct-3-yl)-2,3-dihydro-5-chloroindole-1-carboxamide,
endo-N-(8-methyl-8-azabicyclo[3.2.1]oct-3-yl)-2,3-dihydro-5-methoxyindole-1-carboxamide,
endo-N-(8-methyl-8-azabicyclo[3.2.1]oct-3-yl)indole-1-carboxamide,
N-(1-azabicyclo[2.2.2]oct-3-yl)2,3-dihydroindole-1-carboxamide,
endo-N-(8-methyl-8-azabicyclo[3.2.1]oct-3-yl)-2,3-dihydro-3-ethylindole-1-carboxamide,
endo-N-(8-methyl-8-azabicyclo[3.2.1]oct-3-yl)-3-ethylindole-1-carboxamide,
endo-N-(8-methyl-8-azabicyclo[3.2.1]oct-3-yl)-2,3-dihydro-5-nitroindole-1-carboxamide,
endo-N-(8-methyl-8-azabicyclo[3.2.1]oct-3-yl)-2,3-dihydro-6-nitroindole-1-carboxamide,
N-(1-azabicyclo[2.2.2]oct-3-yl)2,3-dihydro-3,3-dimethylindole-1-carboxamide, or a pharmaceutically acceptable salt of any of the foregoing.

10. endo-N-(8-Methyl-8-azabicyclo[3.2.1]oct-3-yl)-2,3-dihydro-3,3-dimethylindole-1-carboxamide or a pharmaceutically acceptable salt thereof.

11. A process for the preparation of a compound of formula (I) according to claim 1, or a pharmaceutically acceptable salt thereof, which process comprises reacting a compound of formula (V): 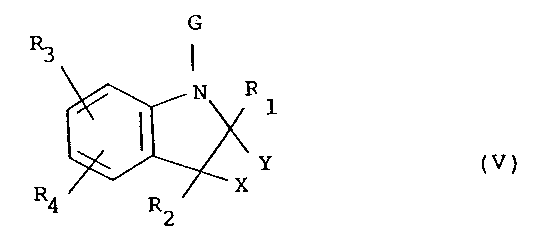 with a compound of formula (VI):
J-Z¹ (VI)
wherein
G is COQ₁ where Q₁ is a leaving group, or hydrogen; and, when G is COQ₁, J is NH₂, or OH or a reactive derivative thereof or, when G is hydrogen, J is a group containing an activated carbonyl group capable of forming a CO-L-linkage with the compound of formula (V); Z¹ is Z as defined or wherein R₅/R₆ is replaced by a hydrogenolysable protecting group; and the remaining variables are as defined in claim 1; and thereafter optionally converting any R₃ and R₄ group to another R₃ and R₄ group respectively, converting Z¹, when other than Z, to Z; converting X and Y to other X and Y; and optionally forming a pharmaceutically acceptable salt of the resultant compound of formula (I).

12. A compound of formula (V) as defined in claim 11, wherein G is COQ₁, excluding 1-(2,3-dihydro) indolyl carbonyl chloride and excluding compounds of formula (V) in which Q₁ is methoxy.

13. 1-(2,3-Dihydro)-indolyltrichloromethyl carbamate,
1-(2,3-dihydro-3-methyl)indolyl-O-(1-succinimidyl)carbamate,
1-(2,3-dihydro-5-fluoro)indolyl-O-(1-succinimidyl)carbamate,
1-(2,3-dihydro-5-methoxy)indolyl trichloromethyl carbamate,
1-(2,3-dihydro-3-ethyl)indolylcarbonyl chloride,
1-(2,3-dihydro-5-nitro)indolyl-trichloromethyl carbamate,
1-[1-(2,3-dihydro-6-nitro)indolylcarbonyl]-imidazole, or
1-(2,3-dihydro-3,3-dimethyl)indolylcarbonyl chloride.

14. A pharmaceutical composition comprising a compound according to any one of claims 1 to 10, and a pharmaceutically acceptable carrier.

15. A compound according to any one of claims 1 to 10, for use as an active therapeutic substance.

16. A compound according to any one of claims 1 to 10 for use in the treatment of migraine, cluster headache and/or trigeminal neuralgia.

17. A compound according to any one of claims 1 to 10 for use in the treatment of emesis including cytotoxic agent or radiation induced nausea and vomiting.

18. A compound according to any one of claims 1 to 10 for use in the treatment of CNS disorders.

19. A compound according to any one of claims 1 to 10 for use in the treatment of anxiety.

20. A compound according to any one of claims 1 to 10 for use in the treatment of psychosis.

21. A compound according to any one of claims 1 to 10 for use in the treatment of arrhythmia.

22. A compound according to any one of claims 1 to 10 for use in the treatment of obesity.

23. A compound according to any one of claims 1 to 10 for use in the treatment of irritable bowel syndrome.

24. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of migraine, cluster headache and/or trigeminal neuralgia.

25. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of emesis including cytotoxic agent or radiation induced nausea and vomiting.

26. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of CNS disorders.

27. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of anxiety.

28. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of psychosis.

29. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of arrhythmia.

30. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of obesity.

31. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of irritable bowel syndrome.

## Claims (Claims for the following Contracting State(s): ES)

1. A process for the preparation of a compound of formula (I), or a pharmaceutically acceptable salt thereof: 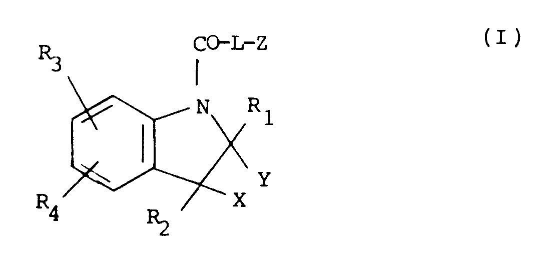 wherein
L is NH or O;
X and Y are independently selected from hydrogen or C₁₋₄ alkyl, or together are a bond;
R₁ and R₂ are independently selected from hydrogen, C₁₋₆ alkyl, C₂₋₆ alkenyl-C₁₋₄ alkyl, or together are C₂₋₄ polymethylene;
R₃ and R₄ are independently selected from hydrogen, halogen, CF₃, C₁₋₆ alkyl, C₁₋₆ alkoxy, C₁₋₆ alkylthio, C₁₋₇ acyl, C₁₋₇ acylamino, C₁₋₆ alkylsulphonylamino, N-(C₁₋₆ alkylsulphonyl)-N-C₁₋₄ alkylamino, C₁₋₆ alkylsulphinyl, hydroxy, nitro or amino, aminocarbonyl, aminosulphonyl, aminosulphonylamino or N-(aminosulphonyl)-C₁₋₄ alkylamino optionally N-substituted by one or two groups selected from C₁₋₆ alkyl, C₃₋₈ cycloalkyl, C₃₋₈ cycloalkyl C₁₋₄ alkyl, phenyl or phenyl C₁₋₄ alkyl groups or optionally N-disubstituted by C₄₋₅ polymethylene;
Z is a group of formula (a), (b) or (c) 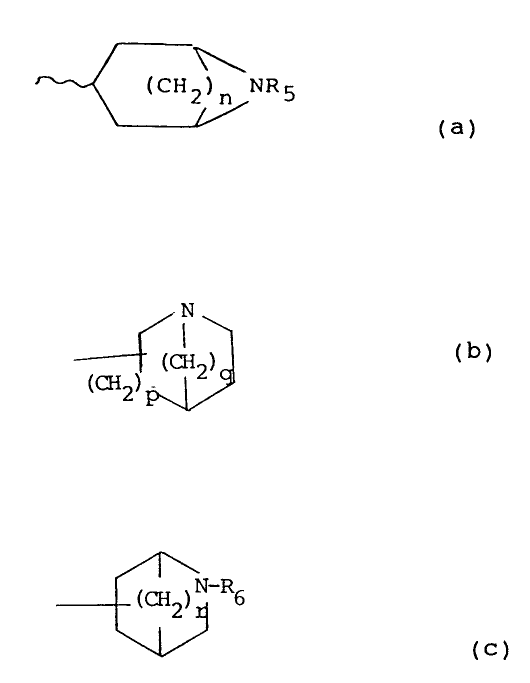 wherein n is 2 or 3; p is 1 or 2; q is 1 to 3; r is 1 to 3; and
R₅ or R₆ is C₁₋₇ alkyl, C₃₋₈ cycloalkyl, C₃₋₈ cycloalkyl-C₁₋₂ alkyl or C₂₋₇ alkenyl-C₁₋₄ alkyl; with the proviso that the compound of formula (I) is other than 2,3-dihydroindole-1-carboxylic acid 1-azabicyclo[2.2.2]oct-3-yl ester;
which process comprises reacting a compound of formula (V): 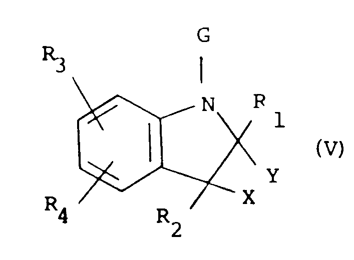 with a compound of formula (VI):
J-Z¹ (VI)
wherein
G is COQ₁ where Q₁ is a leaving group, or hydrogen, and, when G is COQ₁, J is NH₂, or OH or a reactive derivative thereof or, when G is hydrogen, J is a group containing an activated carbonyl group capable of forming a CO-L-linkage with the compound of formula (V); Z¹ is Z as defined or wherein R₅/R₆ is replaced by a hydrogenolysable protecting group; and the remaining variables are as defined in claim 1; and thereafter optionally converting any R₃ and R₄ group to another R₃ and R₄ group respectively, converting Z¹, when other than Z, to Z; converting X and Y to other X and Y; and optionally forming a pharmaceutically acceptable salt of the resultant compound of formula (I).

2. A process for the preparation of a compound according to claim 1 of formula (II): 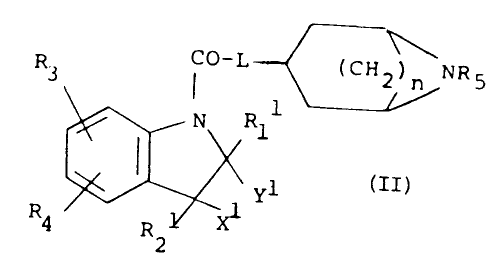 wherein X¹ and Y¹ are independently hydrogen, methyl or ethyl, or together are a bond, R₁¹ and R₂¹ are independently hydrogen, methyl or ethyl and the remaining variables are as defined in claim 1.

3. A process according to claim 2 wherein n is 2.

4. A process according to claim 2 or 3 wherein R₅ is methyl.

5. A process for the preparation of a compound according to claim 1 of formula (III): 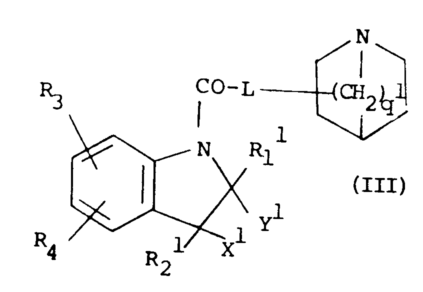 wherein q¹ is 1 or 2 and the remaining variables are as defined in claims 1 and 2.

6. A process according to claim 5 wherein q¹ is 2.

7. A process according to any one of claims 1 to 6 wherein one of R₃ and R₄ is hydrogen and the other is selected from hydrogen, 5-chloro and 5-fluoro.

8. A process according to any one of claims 1 to 7 wherein L is NH.

9. A process according to claim 1 for the preparation of:
endo-N-(9-Methyl-9-azabicyclo[3.3.1]non-3-yl) -2,3-dihydroindole-1-carboxamide,
endo-N-(8-methyl-8-azabicyclo[3.2.1]oct-3-yl) -2,3-dihydroindole-1-carboxamide,
endo(8-methyl-8-azabicyclo[3,2,1]oct-3-yl)-2,3-dihydroindole carboxylic acid ester,
endo-N-(8-methyl-8-azabicyclo[3.2.1]oct-3-yl) -2,3-dihydro-3-methylindole-1-carboxamide,
endo-N-(8-methyl-8-azabicyclo[3,2,1]oct-3-yl) -3-methyl-indole-1-carboxamide,
endo-N-(8-ethyl-8-azabicyclo[3.2.1]oct-3-yl) -2,3-dihydroindole-1-carboxamide,
endo-N-(8-methyl-8-azabicyclo[3.2.1]oct-3-yl) -5-fluoro-2,3-dihydroindole-1-carboxamide,
endo-N-(8-methyl-8-azabicyclo[3.2.1]oct-3-yl)-2,3-dihydro-5-chloroindole-1-carboxamide,
endo-N-(8-methyl-8-azabicyclo[3.2.1]oct-3-yl)-2,3-dihydro-5-methoxyindole-1-carboxamide,
endo-N-(8-methyl-8-azabicyclo[3.2.1]oct-3-yl)indole-1-carboxamide,
N-(1-azabicyclo[2.2.2]oct-3-yl)-2,3-dihydroindole-1-carboxylic acid ester,
endo-N-(8-methyl-8-azabicyclo[3.2.1]oct-3-yl)-2,3-dihydro-3-ethylindole-1-carboxamide,
endo-N-(8-methyl-8-azabicyclo[3.2.1]oct-3-yl)-3-ethyl-indole-1-carboxamide,
endo-N-(8-methyl-8-azabicyclo[3.2.1]oct-3-yl)-2,3-dihydro-5-nitroindole-1-carboxamide,
endo-N-(8-methyl-8-azabicyclo[3.2.1]oct-3-yl)-2,3-dihydro-6-nitroindole-1-carboxamide,
N-(1-azabicyclo[2.2.2]oct-3-yl)2,3-dihydro-3,3-dimethylindole-1-carboxamide, or a pharmaceutically acceptable salt of any of the foregoing.

10. A process according to claim 1 for the preparation of endo-N-(8-Methyl-8-azabicyclo[3.2.1]oct-3-yl)-2,3-dihydro-3,3-dimethylindole-1-carboxamide or a pharmaceutically acceptable salt thereof.

11. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of migraine, cluster headache and/or trigeminal neuralgia.

12. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of emesis including cytotoxic agent or radiation induced nausea and vomiting.

13. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of CNS disorders.

14. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of anxiety.

15. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of psychosis.

16. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of arrhythmia.

17. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of obesity.

18. Use of a compound of formula (I) as defined in any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of irritable bowel syndrome.

## Claims (Claims for the following Contracting State(s): GR)

1. A compound of formula (I), or a pharmaceutically acceptable salt thereof: 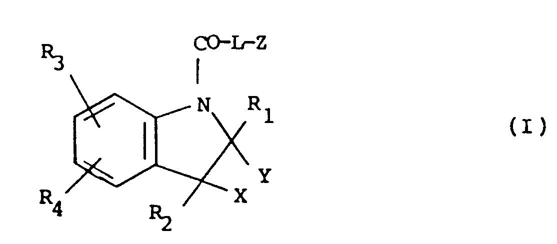 wherein
L is NH or O;
X and Y are independently selected from hydrogen or C₁₋₄ alkyl, or together are a bond;
R₁ and R₂ are independently selected from hydrogen, C₁₋₆ alkyl, C₂₋₆ alkenyl-C₁₋₄ alkyl, or together are C₂₋₄ polymethylene;
R₃ and R₄ are independently selected from hydrogen, halogen, CF₃, C₁₋₆ alkyl, C₁₋₆ alkoxy, C₁₋₆ alkylthio, C₁₋₇ acyl, C₁₋₇ acylamino, C₁₋₆ alkylsulphonylamino, N-(C₁₋₆ alkylsulphonyl)-N-C₁₋₄ alkylamino, C₁₋₆ alkylsulphinyl, hydroxy, nitro or amino, aminocarbonyl, aminosulphonyl, aminosulphonylamino or N-(aminosulphonyl)-C₁₋₄ alkylamino optionally N-substituted by one or two groups selected from C₁₋₆ alkyl, C₃₋₈ cycloalkyl, C₃₋₈ cycloalkyl C₁₋₄ alkyl, phenyl or phenyl C₁₋₄ alkyl groups or optionally N-disubstituted by C₄₋₅ polymethylene;
Z is a group of formula (a), (b) or (c) 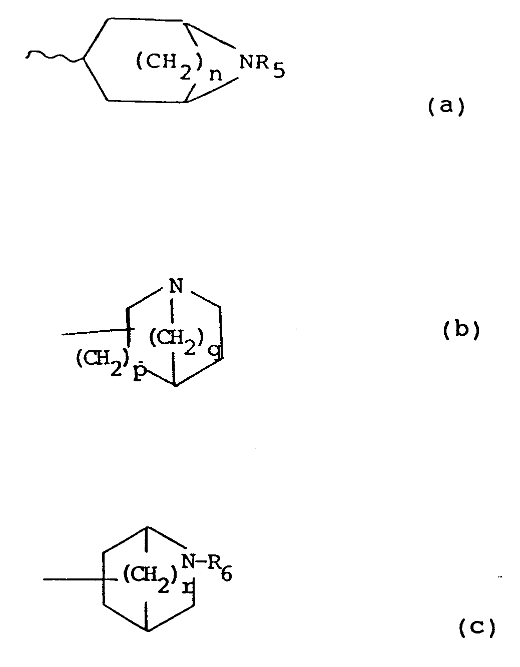 wherein n is 2 or 3; p is 1 or 2; q is 1 to 3; r is 1 to 3; and
R₅ or R₆ is C₁₋₇ alkyl, C₃₋₈ cycloalkyl, C₃₋₈ cycloalkyl-C₁₋₂ alkyl or C₂₋₇ alkenyl-C₁₋₄ alkyl; with the proviso that the compound of formula (I) is other than 2,3-dihydroindole-1-carboxylic acid 1-azabicyclo[2.2.2]oct-3-yl ester;

2. A compound according to claim 1 of formula (II): 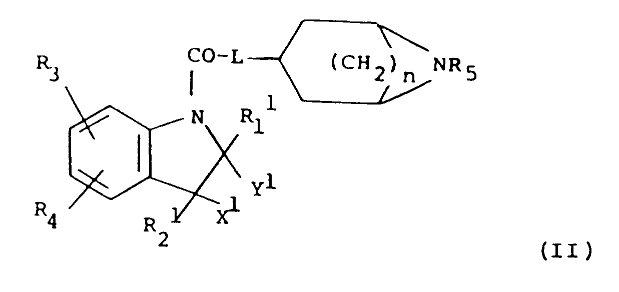 wherein X¹ and Y¹ are independently hydrogen, methyl or ethyl, or together are a bond, R₁¹ and R₂¹ are independently hydrogen, methyl or ethyl and the remaining variables are as defined in claim 1.

3. A compound according to claim 2 wherein n is 2.

4. A compound according to claim 2 or 3 wherein R₅ is methyl.

5. A compound according to claim 1 of formula (III): 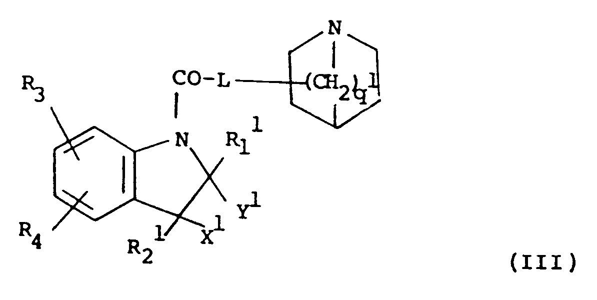 wherein q¹ is 1 or 2 and the remaining variables are as defined in claims 1 and 2.

6. A compound according to claim 5 wherein q¹ is 2.

7. A compound according to any one of claims 1 to 6 wherein one of R₃ and R₄ is hydrogen and the other is selected from hydrogen, 5-chloro and 5-fluoro.

8. A compound according to any one of claims 1 to 7 wherein L is NH.

9. endo-N-(9-Methyl-9-azabicyclo[3.3.1]non-3-yl) -2,3-dihydroindole-1-carboxamide,
endo-N-(8-methyl-8-azabicyclo[3.2.1]oct-3-yl) -2,3-dihydroindole-1-carboxamide,
endo(8-methyl-8-azabicyclo[3,2,1]oct-3-yl)-2,3-dihydroindole carboxylic acid ester,
endo-N-(8-methyl-8-azabicyclo[3.2.1]oct-3-yl) -2,3-dihydro-3-methylindole-1-carboxamide,
endo-N-(8-methyl-8-azabicyclo[3,2,1]oct-3-yl) -3-methyl-indole-1-carboxamide,
endo-N-(8-ethyl-8-azabicyclo[3.2.1]oct-3-yl) -2,3-dihydroindole-1-carboxamide,
endo-N-(8-methyl-8-azabicyclo[3.2.1]oct-3-yl) -5-fluoro-2,3-dihydroindole-1-carboxamide,
endo-N-(8-methyl-8-azabicyclo[3.2.1]oct-3-yl)-2,3-dihydro-5-chloroindole-1-carboxamide,
endo-N-(8-methyl-8-azabicyclo[3.2.1]oct-3-yl)-2,3-dihydro-5-methoxyindole-1-carboxamide,
endo-N-(8-methyl-8-azabicyclo[3.2.1]oct-3-yl)-indole-1-carboxamide,
N-(1-azabicyclo[2.2.2]oct-3-yl)2,3-dihydroindole-1-carboxamide,
endo-N-(8-methyl-8-azabicyclo[3.2.1]oct-3-yl)-2,3-dihydro-3-ethylindole-1-carboxamide,
endo-N-(8-methyl-8-azabicyclo[3.2.1]oct-3-yl)-3-ethylindole-1-carboxamide,
endo-N-(8-methyl-8-azabicyclo[3.2.1]oct-3-yl)-2,3-dihydro-5-nitroindole-1-carboxamide,
endo-N-(8-methyl-8-azabicyclo[3.2.1]oct-3-yl)-2,3-dihydro-6-nitroindole-1-carboxamide,
N-(1-azabicyclo[2.2.2]oct-3-yl)2,3-dihydro-3,3-dimethylindole-1-carboxamide, or a pharmaceutically acceptable salt of any of the foregoing.

10. endo-N-(8-Methyl-8-azabicyclo[3.2.1]oct-3-yl)-2,3-dihydro-3,3-dimethylindole-1-carboxamide or a pharmaceutically acceptable salt thereof.

11. A process for the preparation of a compound of formula (I) according to claim 1, or a pharmaceutically acceptable salt thereof, which process comprises reacting a compound of formula (V): 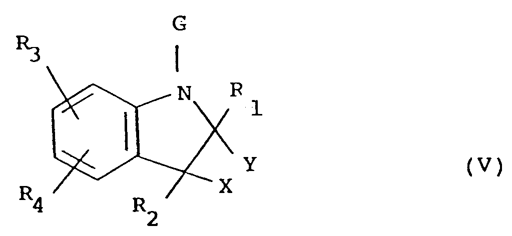 with a compound of formula (VI):
J-Z¹ (VI)
wherein
G is COQ₁ where Q₁ is a leaving group, or hydrogen; and, when G is COQ₁, J is NH₂, or OH or a reactive derivative thereof or, when G is hydrogen, J is a group containing an activated carbonyl group capable of forming a CO-L-linkage with the compound of formula (V); Z¹ is Z as defined or wherein R₅/R₆ is replaced by a hydrogenolysable protecting group; and the remaining variables are as defined in claim 1; and thereafter optionally converting any R₃ and R₄ group to another R₃ and R₄ group respectively, converting Z¹, when other than Z, to Z; converting X and Y to other X and Y; and optionally forming a pharmaceutically acceptable salt of the resultant compound of formula (I).

12. A compound of formula (V) as defined in claim 11, wherein G is COQ₁, excluding 1-(2,3-dihydro) indolyl carbonyl chloride and excluding compounds of formula (V) in which Q₁ is methoxy.

13. 1-(2,3-Dihydro)-indolyltrichloromethyl carbamate,
1-(2,3-dihydro-3-methyl)indolyl-O-(1-succinimidyl)carbamate,
1-(2,3-dihydro-5-fluoro)indolyl-O-(1-succinimidyl)carbamate,
1-(2,3-dihydro-5-methoxy)indolyl trichloromethyl carbamate,
1-(2,3-dihydro-3-ethyl)indolylcarbonyl chloride,
1-(2,3-dihydro-5-nitro)indolyl-trichloromethyl carbamate,
1-[1-(2,3-dihydro-6-nitro)indolylcarbonyl]-imidazole, or
1-(2,3-dihydro-3,3-dimethyl)indolylcarbonyl chloride.

14. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of migraine, cluster headache and/or trigeminal neuralgia.

15. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of emesis including cytotoxic agent or radiation induced nausea and vomiting.

16. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of CNS disorders.

17. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of anxiety.

18. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of psychosis.

19. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of arrhythmia.

20. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of obesity.

21. Use of a compound according to any one of claims 1 to 10 or 2,3-dihydroindole-1-carboxylic acid 1-azabicyclo[2.2.2]oct-3-yl ester, or a pharmaceutically acceptable salt thereof, in the preparation of a medicament for the treatment of irritable bowel syndrome.

## Patentansprüche (Patentansprüche für folgende(n) Vertragsstaat(en): BE, CH, DE, FR, GB, IT, LI, LU, NL, SE)

1. Verbindung der Formel (I) oder deren pharmazeutisch verträgliches Salz: 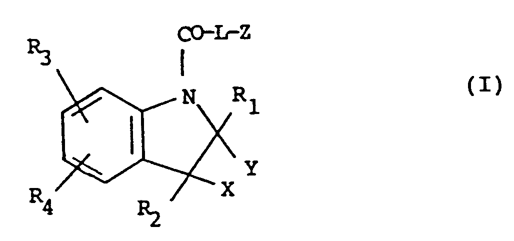 in der
L eine Gruppe NH oder O ist;
X und Y unabhängig voneinander ein Wasserstoffatom oder ein C₁-₄-Alkylrest sind, oder zusammen eine Bindung darstellen;
R₁ und R₂ unabhängig voneinander ein Wasserstoffatom, ein C₁₋₆-Alkyl- oder ein C₂₋₆-Alkenyl-C₁₋₄-alkylrest sind oder zusammen einen C₂₋₄-Polymethylenrest darstellen;
R₃ und R₄ unabhängig voneinander ein Wasserstoff- oder Halogenatom, einen Trifluormethyl-, C₁₋₆-Alkyl-, C₁₋₆-Alkoxy-, C₁₋₆-Alkylthio-, C₁₋₇-Acyl-, C₁₋₇-Acylamino-, C₁₋₆-Alkylsulfonylamino-, N-(C₁₋₆-Alkylsulfonyl)-N-C₁₋₄-alkylamino- oder C₁₋₆-Alkylsulfinylrest, eine Hydroxy-, Nitro- oder Aminogruppe, einen Aminocarbonyl-, Aminosulfonyl-, Aminosulfonylamino- oder N-(Aminosulfonyl)-C₁₋₄-alkylaminorest bedeuten, gegebenenfalls N-substituiert durch eine oder zwei Gruppen, ausgewählt aus C₁₋₆-Alkyl-, C₃₋₈-Cycloalkyl-, C₃₋₈-Cycloalkyl-C₁₋₄-alkyl-, Phenyl- oder Phenyl-C₁₋₄-alkylresten, oder gegebenenfalls N-disubstituiert durch einen C₄₋₅-Polymethylenrest;
Z ein Rest der Formeln (a), (b) oder (c) ist 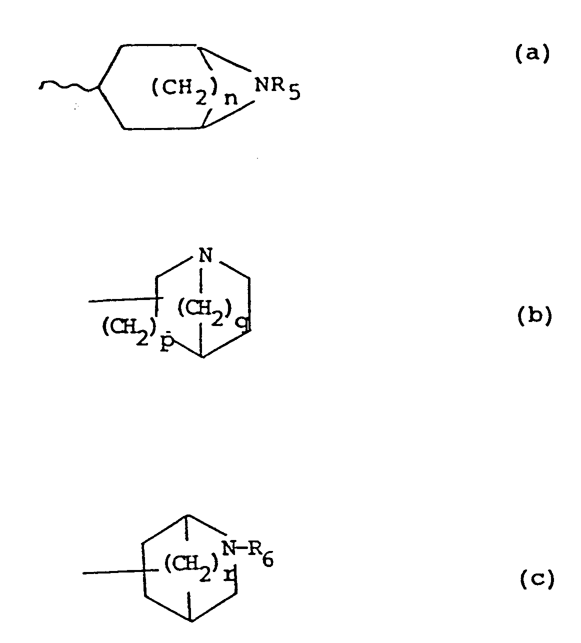 in denen n 2 oder 3 ist; p 1 oder 2 ist; q 1 bis 3 ist; r 1 bis 3 ist; und
R₅ oder R₆ ein C₁₋₇-Alkyl-, C₃₋₈-Cycloalkyl-, C₃₋₈-Cycloalkyl-C₁₋₂-alkyl- oder C₂₋₇-Alkenyl-C₁₋₄-alkylrest sind; mit der Maßgabe, daß die Verbindung der Formel (I) nicht 2,3-Dihydroindol-1-carbonsäure-1-azabicyclo[2.2.2]oct-3-ylester ist.

2. Verbindung gemäß Anspruch 1 der Formel (II) 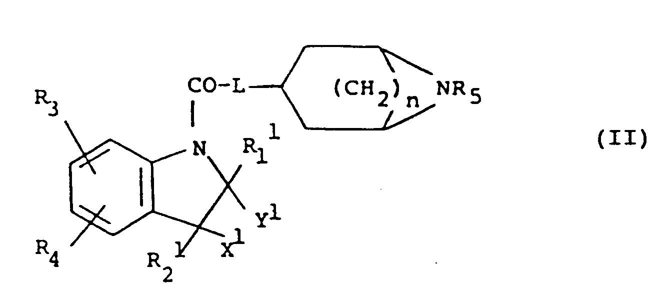 in der X¹ und Y¹ unabhängig voneinander ein Wasserstoffatom, eine Methyl- oder Ethylgruppe oder zusammen eine Bindung bedeuten, R₁¹ und R₂¹ unabhängig voneinander ein Wasserstoffatom, eine Methyl- oder Ethylgruppe bedeuten und die restlichen Variablen wie in Anspruch 1 definiert sind.

3. Verbindung gemäß Anspruch 2, in der n 2 ist.

4. Verbindung gemäß Anspruch 2 oder 3, in der R₅ eine Methylgruppe ist.

5. Verbindung gemäß Anspruch 1 der Formel (III): 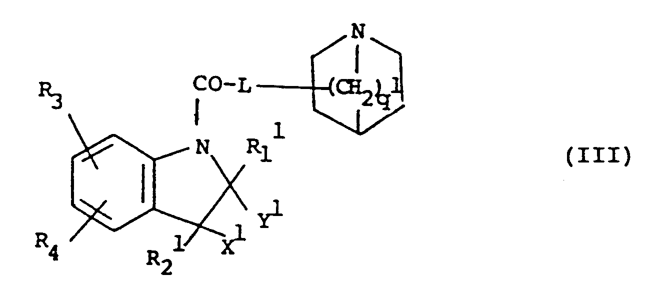 in der q¹ 1 oder 2 ist und die restlichen Variablen wie in den Ansprüchen 1 und 2 definiert sind.

6. Verbindung gemäß Anspruch 5, in der q¹ 2 ist.

7. Verbindung gemäß einem der Ansprüche 1 bis 6, in der einer der Reste R₃ und R₄ ein Wasserstoffatom ist und der andere ausgewählt ist aus einem Wasserstoff-, 5-Chlor- und 5-Fluoratom.

8. Verbindung gemäß einem der Ansprüche 1 bis 7, in der L eine Gruppe NH ist.

9. endo-N-(9-Methyl-9-azabicyclo[3.3.1]non-3-yl)-2,3-dihydroindol-1-carbonsäureamid,
endo-N-(8-Methyl-8-azabicyclo[3.2.1]oct-3-yl)-2,3-dihydroindol-1-carbonsäureamid,
endo(8-Methyl-8-azabicyclo[3.2.1]oct-3-yl)-2,3-dihydroindolcarbonsäureester,
endo-N-(8-Methyl-8-azabicyclo[3.2.1]oct-3-yl)-2,3-dihydro-3-methylindol-1-carbonsäureamid,
endo-N-(8-Methyl-8-azabicyclo[3.2.1]oct-3-yl)-3-methylindol-1-carbonsäureamid,
endo-N-(8-Ethyl-8-azabicyclo[3.2.1]oct-3-yl)-2,3-dihydroindol-1-carbonsäureamid,
endo-N-(8-Methyl-8-azabicyclo[3.2.1]oct-3-yl)-5-fluor-2,3-dihydroindol-1-carbonsäureamid,
endo-N-(8-Methyl-8-azabicyclo[3.2.1]oct-3-yl)-2,3-dihydro-5-chlorindol-1-carbonsäureamid,
endo-N-(8-Methyl-8-azabicyclo[3.2.1]oct-3-yl)-2,3-dihydro-5-methoxyindol-1-carbonsäureamid,
endo-N-(8-Methyl-8-azabicyclo[3.2.1]oct-3-yl)-indol-1-carbonsäureamid,
N-(1-Azabicyclo[2.2.2]oct-3-yl)-2,3-dihydroindol-1-carbonsäureamid,
endo-N-(8-Methyl-8-azabicyclo[3.2.1]oct-3-yl)-2,3-dihydro-3-ethylindol-1-carbonsäureamid,
endo-N-(8-Methyl-8-azabicyclo[3.2.1]oct-3-yl)-3-ethylindol-1-carbonsäureamid,
endo-N-(8-Methyl-8-azabicyclo[3.2.1]oct-3-yl)-2,3-dihydro-5-nitroindol-1-carbonsäureamid,
endo-N-(8-Methyl-8-azabicyclo[3.2.1]oct-3-yl)-2,3-dihydro-6-nitroindol-1-carbonsäureamid,
N-(1-Azabicyclo[2.2.2]oct-3-yl)2,3-dihydro-3,3-dimethylindol-1-carbonsäureamid, oder deren pharmazeutisch verträgliche Salze.

10. endo-N-(8-Methyl-8-azabicyclo[3.2.1]oct-3-yl)-2,3-dihydro-3,3-dimethylindol-1-carbonsäureamid oder dessen pharmazeutisch verträgliches Salz.

11. Verfahren zur Herstellung einer Verbindung der Formel (I) gemäß Anspruch 1 oder eines pharmazeutisch verträglichen Salzes davon, umfassend die Umsetzung einer Verbindung der Formel (V): 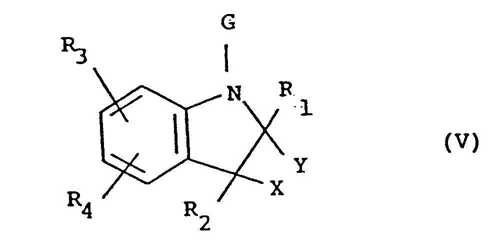 mit einer Verbindung der Formel (VI)
J-Z¹ (VI)
in denen
G eine Gruppe COQ₁ ist, in der Q₁ eine Abgangsgruppe oder ein Wasserstoffatom bedeutet; und
wenn G eine Gruppe COQ₁ ist, J eine Amino- oder Hydroxygruppe oder ein reaktives Derivat davon bedeutet; oder wenn G ein Wasserstoffatom ist, J eine eine aktivierte Carbonylgruppe enthaltende Gruppe bedeutet, die fähig ist zum Ausbilden einer CO-L-Bindung mit der Verbindung der Formel (V);
Z¹ eine Gruppe Z ist, die die vorstehend angegebene Bedeutung hat oder in der R₅/R₆ durch eine hydrogenolysierbare Schutzgruppe ersetzt sind;
und die restlichen Variablen die in Anspruch 1 angegebene Bedeutung haben; und
gegebenenfalls anschließend das Umwandeln der Reste R₃ bzw. R₄ in andere Reste R₃ bzw. R₄, das Umwandeln von Z¹ in Z, wenn Z¹ verschieden von Z ist; das Umwandeln der Reste X und Y in andere Reste X und Y; und gegebenenfalls die Bildung eines pharmazeutisch verträglichen Salzes der resultierenden Verbindung der Formel (I).

12. Verbindung der Formel (V) gemäß Anspruch 11, in der G ein Rest COQ₁ ist, ausgenommen 1-(2,3-Dihydro)-indolylcarbonsäurechlorid und Verbindungen der Formel (V), in denen Q₁ eine Methoxygruppe ist.

13. 1-(2,3-Dihydro)-indolyltrichlormethylcarbamat,
1-(2,3-Dihydro-3-methyl)indolyl-O-(1-succinimidyl)carbamat,
1-(2,3-Dihydro-5-fluor)indolyl-O-(1-succinimidyl)carbamat,
1-(2,3-Dihydro-5-methoxy)indolyltrichlormethylcarbamat,
1-(2,3-Dihydro-3-ethyl)indolylcarbonylsäurechlorid,
1-(2,3-Dihydro-5-nitro(indolyl-trichlormethylcarbamat,
1-[1-(2,3-Dihydro-6-nitro)indolylcarbonyl]imidazol, oder
1-(2,3-Dihydro-3,3-dimethyl)indolylcarbonsäurechlorid.

14. Arzneimittel, umfassend eine Verbindung gemäß einem der Ansprüche 1 bis 10 und einen pharmazeutisch verträglichen Träger.

15. Verbindung gemäß einem der Ansprüche 1 bis 10 zur Verwendung als therapeutischer Wirkstoff.

16. Verbindung gemäß einem der Ansprüche 1 bis 10, zur Verwendung bei der Behandlung von Migräne, clusterartigem Kopfschmerz und/oder trigeminaler Neuralgie.

17. Verbindung gemäß einem der Ansprüche 1 bis 10 zur Verwendung bei der Behandlung von Erbrechen, einschließlich durch ein cytotoxisches Mittel oder Bestrahlung induzierten Brechreiz und Erbrechen.

18. Verbindung gemäß einem der Ansprüche 1 bis 10 zur Verwendung bei der Behandlung von Störungen des zentralen Nervensystems.

19. Verbindung gemäß einem der Ansprüche 1 bis 10 zur Verwendung bei der Behandlung von Angstzuständen.

20. Verbindung gemäß einem der Ansprüche 1 bis 10 zur Verwendung bei der Behandlung von Psychosen.

21. Verbindung gemäß einem der Ansprüche 1 bis 10 zur Verwendung bei der Behandlung von Rhythmusstörungen.

22. Verbindung gemäß einem der Ansprüche 1 bis 10 zur Verwendung bei der Behandlung von Fettsucht.

23. Verbindung gemäß einem der Ansprüche 1 bis 10 zur Verwendung bei der Behandlung eines Reizmagens.

24. Verwendung einer Verbindung gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon zur Herstellung eines Medikaments zur Behandlung von Migräne, clusterartigem Kopfschmerz und/oder Trigeminus-Neuralgie.

25. Verwendung einer Verbindung gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon, zur Herstellung eines Medikaments zur Behandlung von Erbrechen, einschließlich durch ein cytotoxisches Mittel oder Bestrahlung induziertem Brechreiz und Erbrechen.

26. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung von Störungen des zentralen Nervensystems.

27. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Angstzuständen.

28. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Psychosen.

29. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Rhythmusstörungen.

30. Verwendung einer Verbindung gemäß einem der Ansprüche 1 bis 10 oder von 2,3-Dihydroindol-1-carbonsäure-1-azabicyclo[2.2.2]oct-3-ylesters, oder eines pharmazeutisch verträglichen Salzes davon zur Herstellung eines Medikaments zur Behandlung von Fettsucht.

31. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung eines Reizmagens.

## Patentansprüche (Patentansprüche für folgende(n) Vertragsstaat(en): ES)

1. Verfahren zur Herstellung einer Verbindung der Formel (I) oder deren pharmazeutisch verträglichem Salz: 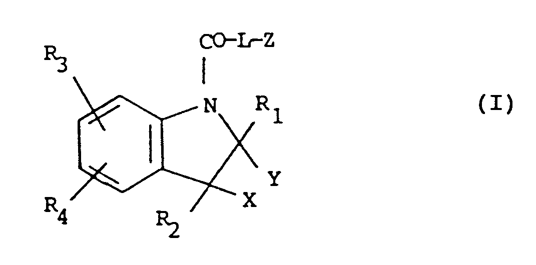 in der
L eine Gruppe NH oder O ist;
X und Y unabhängig voneinander ein Wasserstoffatom oder ein C₁₋₄-Alkylrest sind, oder zusammen eine Bindung darstellen;
R₁ und R₂ unabhängig voneinander ein Wasserstoffatom, ein C₁₋₆-Alkyl- oder ein C₂₋₆-Alkenyl-C₁₋₄-alkylrest sind oder zusammen einen C₂₋₄-Polymethylenrest darstellen;
R₃ und R₄ unabhängig voneinander ein Wasserstoff- oder Halogenatom, einen Trifluormethyl-, C₁₋₆-Alkyl-, C₁₋₆-Alkoxy-, C₁₋₆-Alkylthio-, C₁₋₇-Acyl-, C₁₋₇-Acylamino-, C₁₋₆-Alkylsulfonylamino-, N-(C₁₋₆-Alkylsulfonyl)-N-C₁₋₄-alkylamino- oder C₁₋₆-Alkylsulfinylrest, eine Hydroxy-, Nitro- oder Aminogruppe, einen Aminocarbonyl-, Aminosulfonyl-, Aminosulfonylamino- oder N-(Aminosulfonyl)-C₁₋₄-alkylaminorest bedeuten, gegebenenfalls N-substituiert durch eine oder zwei Gruppen, ausgewählt aus C₁₋₆-Alkyl-, C₃₋₈-Cycloalkyl-, C₃₋₈-Cycloalkyl-C₁₋₄-alkyl-, Phenyl- oder Phenyl-C₁₋₄-alkylresten, oder gegebenenfalls N-disubstituiert durch einen C₄₋₅-Polymethylenrest;
Z ein Rest der Formeln (a), (b) oder (c) ist 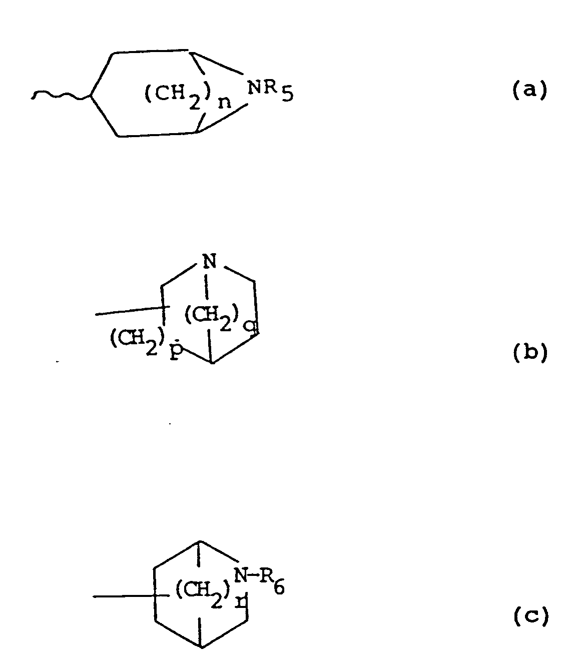 in denen n 2 oder 3 ist; p 1 oder 2 ist; q 1 bis 3 ist; r 1 bis 3 ist; und
R₅ oder R₆ ein C₁₋₇-Alkyl-, C₃₋₈-Cycloalkyl-, C₃₋₈-Cycloalkyl-C₁₋₂-alkyl- oder C₂₋₇-Alkenyl-C₁₋₄-alkylrest sind; mit der Maßgabe, daß die Verbindung der Formel (I) nicht 2,3-Dihydroindol-1-carbonsäure-1-azabicyclo[2.2.2]oct-3-ylester ist, umfassend die Umsetzung einer Verbindung der Formel (V): 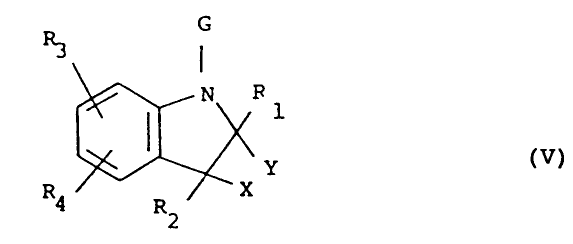 mit einer Verbindung der Formel (VI)
J-Z¹ (VI)
in denen
G eine Gruppe COQ₁ ist, in der Q₁ eine Abgangsgruppe oder ein Wasserstoffatom bedeutet; und
wenn G eine Gruppe COQ₁ ist, J eine Amino- oder Hydroxygruppe oder ein reaktives Derivat davon bedeutet; oder wenn G ein Wasserstoffatom ist, J eine eine aktivierte Carbonylgruppe enthaltende Gruppe bedeutet, die fähig ist zum Ausbilden einer CO-L-Bindung mit der Verbindung der Formel (V);
Z¹ eine Gruppe Z ist, die die vorstehend angegebene Bedeutung hat oder in der R₅/R₆ durch eine hydrogenolysierbare Schutzgruppe ersetzt sind;
und die restlichen Variablen die in Anspruch 1 angegebene Bedeutung haben; und
gegebenenfalls anschließend das Umwandeln der Reste R₃ bzw. R₄ in andere Reste R₃ bzw. R₄, das Umwandeln von Z¹ in Z, wenn Z¹ verschieden von Z ist; das Umwandeln der Reste X und Y in andere Reste X und Y; und gegebenenfalls die Bildung eines pharmazeutisch verträglichen Salzes der resultierenden Verbindung der Formel (I).

2. Verfahren zur Herstellung einer Verbindung gemäß Anspruch 1 der Formel (II) 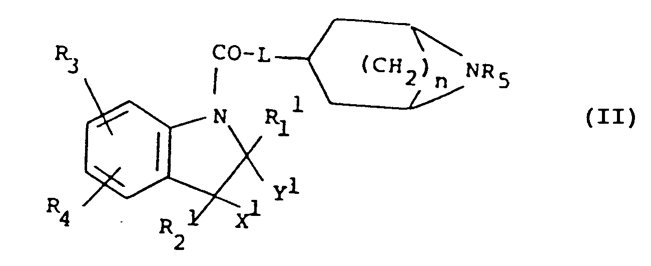 in der X¹ und Y¹ unabhängig voneinander ein Wasserstoffatom, eine Methyl- oder Ethylgruppe oder zusammen eine Bindung bedeuten, R₁¹ und R₂¹ unabhängig voneinander ein Wasserstoffatom, eine Methyl- oder Ethylgruppe bedeuten und die restlichen Variablen wie in Anspruch 1 definiert sind.

3. Verfahren gemäß Anspruch 2, in der n 2 ist.

4. Verfahren gemäß Anspruch 2 oder 3, in der R₅ eine Methylgruppe ist.

5. Verfahren zur Herstellung einer Verbindung gemäß Anspruch 1 der Formel (III): 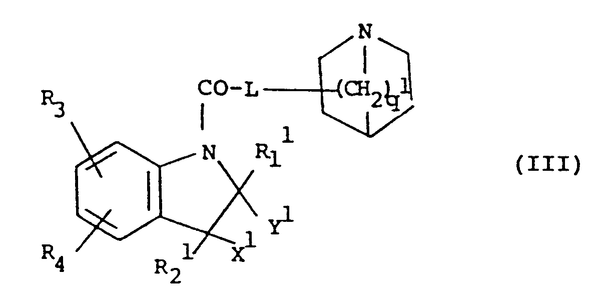 in der q¹ 1 oder 2 ist und die restlichen Variablen wie in den Ansprüchen 1 und 2 definiert sind.

6. Verfahren gemäß Anspruch 5, in der q¹ 2 ist.

7. Verfahren gemäß einem der Ansprüche 1 bis 6, in der einer der Reste R₃ und R₄ ein Wasserstoffatom ist und der andere ausgewählt ist aus einem Wasserstoff-, 5-Chlor- und 5-Fluoratom.

8. Verfahren gemäß einem der Ansprüche 1 bis 7, in der L eine Gruppe NH ist.

9. Verfahren gemäß Anspruch 1 zur Herstellung von:
endo-N-(9-Methyl-9-azabicyclo[3.3.1]non-3-yl)-2,3-dihydroindol-1-carbonsäureamid,
endo-N-(8-Methyl-8-azabicyclo[3.2.1]oct-3-yl)-2,3-dihydroindol-1-carbonsäureamid,
endo(8-Methyl-8-azabicyclo[3.2.1]oct-3-yl)-2,3-dihydroindolcarbonsäureester,
endo-N-(8-Methyl-8-azabicyclo[3.2.1]oct-3-yl)-2,3-dihydro-3-methylindol-1-carbonsäureamid,
endo-N-(8-Methyl-8-azabicyclo[3.2.1]oct-3-yl)-3-methylindol-1-carbonsäureamid,
endo-N-(8-Ethyl-8-azabicyclo[3.2.1]oct-3-yl)-2,3-dihydroindol-1-carbonsäureamid,
endo-N-(8-Methyl-8-azabicyclo[3.2.1]oct-3-yl)-5-fluor-2,3-dihydroindol-1-carbonsäureamid,
endo-N-(8-Methyl-8-azabicyclo[3.2.1]oct-3-yl)-2,3-dihydro-5-chlorindol-1-carbonsäureamid,
endo-N-(8-Methyl-8-azabicyclo[3.2.1]oct-3-yl)-2,3-dihydro-5-methoxyindol-1-carbonsäureamid,
endo-N-(8-Methyl-8-azabicyclo[3.2.1]oct-3-yl)-indol-1-carbonsäureamid,
N-(1-Azabicyclo[2.2.2]oct-3-yl)-2,3-dihydroindol-1-carbonsäureamid,
endo-N-(8-Methyl-8-azabicyclo[3.2.1]oct-3-yl)-2,3-dihydro-3-ethylindol-1-carbonsäureamid,
endo-N-(8-Methyl-8-azabicyclo[3.2.1]oct-3-yl)-3-ethylindol-1-carbonsäureamid,
endo-N-(8-Methyl-8-azabicyclo[3.2.1]oct-3-yl)-2,3-dihydro-5-nitroindol-1-carbonsäureamid,
endo-N-(8-Methyl-8-azabicyclo[3.2.1]oct-3-yl)-2,3-dihydro-6-nitroindol-1-carbonsäureamid,
N-(1-Azabicyclo[2.2.2]oct-3-yl)2,3-dihydro-3,3-dimethylindol-1-carbonsäureamid, oder deren pharmazeutisch verträglichen Salzen.

10. Verfahren gemäß Anspruch 1 zur Herstellung von endo-N-(8-Methyl-8-azabicyclo[3.2.1]oct-3-yl)-2,3-dihydro-3,3-dimethylindol-1-carbonsäureamid oder dessen pharmazeutisch verträglichem Salz.

11. Verwendung einer Verbindung der Formel I gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon zur Herstellung eines Medikaments zur Behandlung von Migräne, clusterartigem Kopfschmerz und/oder Trigeminus-Neuralgie.

12. Verwendung einer Verbindung der Formel I gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon, zur Herstellung eines Medikaments zur Behandlung von Erbrechen, einschließlich durch ein cytotoxisches Mittel oder Bestrahlung induziertem Brechreiz und Erbrechen.

13. Verwendung einer Verbindung der Formel I gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung von Störungen des zentralen Nervensystems.

14. Verwendung einer Verbindung der Formel I gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Angstzuständen.

15. Verwendung einer Verbindung der Formel I gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Psychosen.

16. Verwendung einer Verbindung der Formel I gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Rhythmusstörungen.

17. Verwendung einer Verbindung der Formel I gemäß einem der Ansprüche 1 bis 10 oder von 2,3-Dihydroindol-1-carbonsäure-1-azabicyclo[2.2.2]oct-3-ylesters, oder eines pharmazeutisch verträglichen Salzes davon zur Herstellung eines Medikaments zur Behandlung von Fettsucht.

18. Verwendung einer Verbindung der Formel I gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung eines Reizmagens.

## Patentansprüche (Patentansprüche für folgende(n) Vertragsstaat(en): GR)

1. Verbindung der Formel (I) oder deren pharmazeutisch verträgliches Salz: 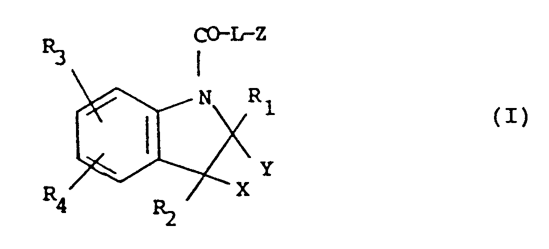 in der
L eine Gruppe NH oder O ist;
X und Y unabhängig voneinander ein Wasserstoffatom oder ein C₁₋₄-Alkylrest sind, oder zusammen eine Bindung darstellen;
R₁ und R₂ unabhängig voneinander ein Wasserstoffatom, ein C₁₋₆-Alkyl- oder ein C₂₋₆-Alkenyl-C₁₋₄-alkylrest sind oder zusammen einen C₂₋₄-Polymethylenrest darstellen;
R₃ und R₄ unabhängig voneinander ein Wasserstoff- oder Halogenatom, einen Trifluormethyl-, C₁₋₆-Alkyl-, C₁₋₆-Alkoxy-, C₁₋₆-Alkylthio-, C₁₋₇-Acyl-, C₁₋₇-Acylamino-, C₁₋₆-Alkylsulfonylamino-, N-(C₁₋₆-Alkylsulfonyl)-N-C₁₋₄-alkylamino- oder C₁₋₆-Alkylsulfinylrest, eine Hydroxy-, Nitro- oder Aminogruppe, einen Aminocarbonyl-, Aminosulfonyl-, Aminosulfonylamino- oder N-(Aminosulfonyl)-C₁₋₄-alkylaminorest bedeuten, gegebenenfalls N-substituiert durch eine oder zwei Gruppen, ausgewählt aus C₁₋₆-Alkyl-, C₃₋₈-Cycloalkyl-, C₃₋₈-Cycloalkyl-C₁₋₄-alkyl-, Phenyl- oder Phenyl-C₁₋₄-alkylresten, oder gegebenenfalls N-disubstituiert durch einen C₄₋₅-Polymethylenrest;
Z ein Rest der Formeln (a), (b) oder (c) ist 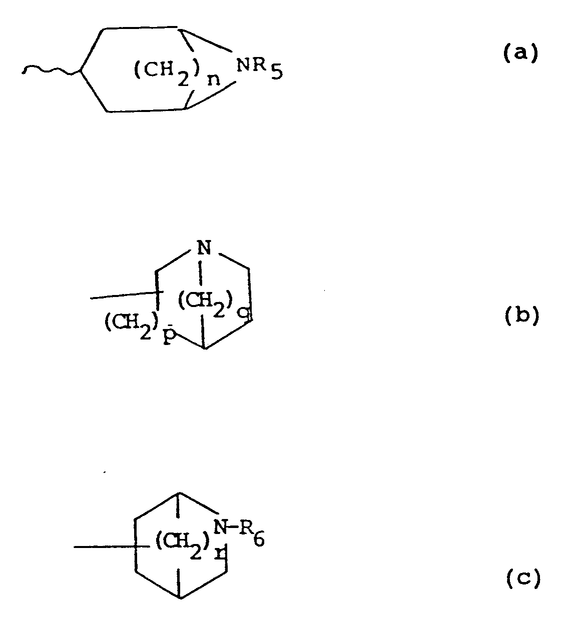 in denen n 2 oder 3 ist; p 1 oder 2 ist; q 1 bis 3 ist; r 1 bis 3 ist; und
R₅ oder R₆ ein C₁₋₇-Alkyl-, C₃₋₈-Cycloalkyl-, C₃₋₈-Cycloalkyl-C₁₋₂-alkyl- oder C₂₋₇-A1keny1-C₁₋₄-alkylrest sind; mit der Maßgabe, daß die Verbindung der Formel (I) nicht 2,3-Dihydroindol-1-carbonsäure-1-azabicyclo[2.2.2]oct-3-ylester ist.

2. Verbindung gemäß Anspruch 1 der Formel (II) 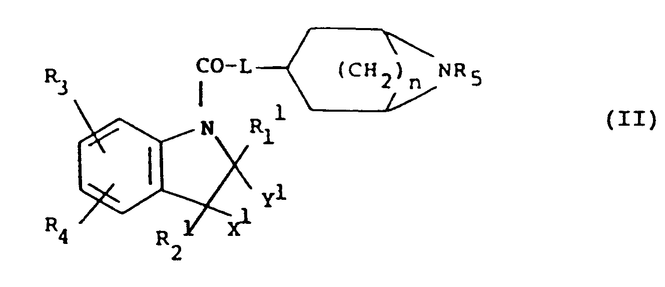 in der X¹ und Y¹ unabhängig voneinander ein Wasserstoffatom, eine Methyl- oder Ethylgruppe oder zusammen eine Bindung bedeuten, R₁¹ und R₂¹ unabhängig voneinander ein Wasserstoffatom, eine Methyl- oder Ethylgruppe bedeuten und die restlichen Variablen wie in Anspruch 1 definiert sind.

3. Verbindung gemäß Anspruch 2, in der n 2 ist.

4. Verbindung gemäß Anspruch 2 oder 3, in der R₅ eine Methylgruppe ist.

5. Verbindung gemäß Anspruch 1 der Formel (III): 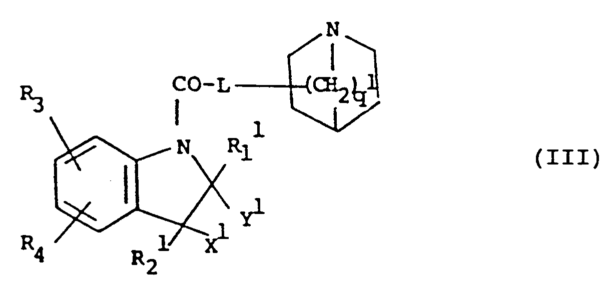 in der q¹ 1 oder 2 ist und die restlichen Variablen wie in den Ansprüchen 1 und 2 definiert sind.

6. Verbindung gemäß Anspruch 5, in der q¹ 2 ist.

7. Verbindung gemäß einem der Ansprüche 1 bis 6, in der einer der Reste R₃ und R₄ ein Wasserstoffatom ist und der andere ausgewählt ist aus einem Wasserstoff-, 5-Chlor- und 5-Fluoratom.

8. Verbindung gemäß einem der Ansprüche 1 bis 7, in der L eine Gruppe NH ist.

9. endo-N-(9-Methyl-9-azabicyclo[3.3.1]non-3-yl)-2,3-dihydroindol-1-carbonsäureamid,
endo-N-(8-Methyl-8-azabicyclo[3.2.1]oct-3-yl)-2,3-dihydroindol-1-carbonsäureamid,
endo(8-Methyl-8-azabicyclo[3.2.1]oct-3-yl)-2,3-dihydroindolcarbonsäureester,
endo-N-(8-Methyl-8-azabicyclo[3.2.1]oct-3-yl)-2,3-dihydro-3-methylindol-1-carbonsäureamid,
endo-N-(8-Methyl-8-azabicyclo[3.2.1]oct-3-yl)-3-methylindol-1-carbonsäureamid,
endo-N-(8-Ethyl-8-azabicyclo[3.2.1]oct-3-yl)-2,3-dihydroindol-1-carbonsäureamid,
endo-N-(8-Methyl-8-azabicyclo[3.2.1]oct-3-yl)-5-fluor-2,3-dihydroindol-1-carbonsäureamid,
endo-N-(8-Methyl-8-azabicyclo[3.2.1]oct-3-yl)-2,3-dihydro-5-chlorindol-1-carbonsäureamid,
endo-N-(8-Methyl-8-azabicyclo[3.2.1]oct-3-yl)-2,3-dihydro-5-methoxyindol-1-carbonsäureamid,
endo-N-(8-Methyl-8-azabicyclo[3.2.1]oct-3-yl)-indol-1-carbonsäureamid,
N-(1-Azabicyclo[2.2.2]oct-3-yl)-2,3-dihydroindol-1-carbonsäureamid,
endo-N-(8-Methyl-8-azabicyclo[3.2.1]oct-3-yl)-2,3-dihydro-3-ethylindol-1-carbonsäureamid,
endo-N-(8-Methyl-8-azabicyclo[3.2.1]oct-3-yl)-3-ethylindol-1-carbonsäureamid,
endo-N-(8-Methyl-8-azabicyclo[3.2.1]oct-3-yl)-2,3-dihydro-5-nitroindol-1-carbonsäureamid,
endo-N-(8-Methyl-8-azabicyclo[3.2.1]oct-3-yl)-2,3-dihydro-6-nitroindol-1-carbonsäureamid,
N-(1-Azabicyclo[2.2.2]oct-3-yl)2,3-dihydro-3,3-dimethylindol-1-carbonsäureamid, oder deren pharmazeutisch verträgliche Salze.

10. endo-N-(8-Methyl-8-azabicyclo[3.2.1]oct-3-yl)-2,3-dihydro-3,3-dimethylindol-1-carbonsäureamid oder dessen pharmazeutisch verträgliches Salz.

11. Verfahren zur Herstellung einer Verbindung der Formel (I) gemäß Anspruch 1 oder eines pharmazeutisch verträglichen Salzes davon, umfassend die Umsetzung einer Verbindung der Formel (V): 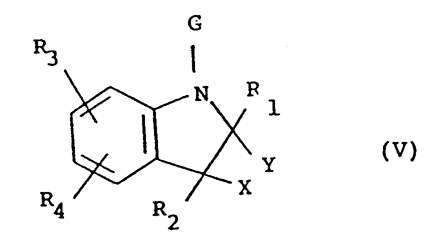 mit einer Verbindung der Formel (VI)
J-Z¹ (VI)
in denen
G eine Gruppe COQ₁ ist, in der Q₁ eine Abgangsgruppe oder ein Wasserstoffatom bedeutet; und
wenn G eine Gruppe COQ₁ ist, J eine Amino- oder Hydroxygruppe oder ein reaktives Derivat davon bedeutet; oder wenn G ein Wasserstoffatom ist, J eine eine aktivierte Carbonylgruppe enthaltende Gruppe bedeutet, die fähig ist zum Ausbilden einer CO-L-Bindung mit der Verbindung der Formel (V);
Z¹ eine Gruppe Z ist, die die vorstehend angegebene Bedeutung hat oder in der R₅/R₆ durch eine hydrogenolysierbare Schutzgruppe ersetzt sind;
und die restlichen Variablen die in Anspruch 1 angegebene Bedeutung haben; und
gegebenenfalls anschließend das Umwandeln der Reste R₃ bzw. R₄ in andere Reste R₃ bzw. R₄, das Umwandeln von Z¹ in Z, wenn Z¹ verschieden von Z ist; das Umwandeln der Reste X und Y in andere Reste X und Y; und gegebenenfalls die Bildung eines pharmazeutisch verträglichen Salzes der resultierenden Verbindung der Formel (I).

12. Verbindung der Formel (V) gemäß Anspruch 11, in der G ein Rest COQ₁ ist, ausgenommen 1-(2,3-Dihydro)-indolylcarbonsäurechlorid und Verbindungen der Formel (V), in denen Q₁ eine Methoxygruppe ist.

13. 1-(2,3-Dihydro)-indolyltrichlormethylcarbamat,
1-(2,3-Dihydro-3-methyl)indolyl-O-(1-succinimidyl)carbamat,
1-(2,3-Dihydro-5-fluor)indolyl-O-(1-succinimidyl)carbamat,
1-(2,3-Dihydro-5-methoxy)indolyltrichlormethylcarbamat,
1-(2,3-Dihydro-3-ethyl)indolylcarbonylsäurechlorid,
1-(2,3-Dihydro-5-nitro(indolyl-trichlormethylcarbamat,
1-[1-(2,3-Dihydro-6-nitro)indolylcarbonyl]imidazol, oder
1-(2,3-Dihydro-3,3-dimethyl)indolylcarbonsäurechlorid.

14. Verwendung einer Verbindung gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon zur Herstellung eines Medikaments zur Behandlung von Migräne, clusterartigem Kopfschmerz und/oder Trigeminus-Neuralgie.

15. Verwendung einer Verbindung gemäß einem der Ansprüche 1 bis 10 oder von 2,3-Dihydroindol-1-carbonsäure-1-azabicyclo[2.2.2]oct-3-ylester oder eines pharmazeutisch verträglichen Salzes davon, zur Herstellung eines Medikaments zur Behandlung von Erbrechen, einschließlich durch ein cytotoxisches Mittel oder Bestrahlung induziertem Brechreiz und Erbrechen.

16. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung von Störungen des zentralen Nervensystems.

17. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Angstzuständen.

18. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Psychosen.

19. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikaments zur Behandlung von Rhythmusstörungen.

20. Verwendung einer Verbindung gemäß einem der Ansprüche 1 bis 10 oder von 2,3-Dihydroindol-1-carbonsäure-1-azabicyclo[2.2.2]oct-3-ylesters, oder eines pharmazeutisch verträglichen Salzes davon zur Herstellung eines Medikaments zur Behandlung von Fettsucht.

21. Verwendung einer Verbindung gemäß einem der Ansprüche 1 bis 10 oder von 2,3-Dihydroindol-1-carbonsäure-1-azabicyclo[2.2.2]oct-3-ylester, oder eines pharmazeutisch verträglichen Salzes davon zur Herstellung eines Medikamentes zur Behandlung eines Reizmagens.

## Revendications (Revendications pour l'(les) Etat(s) contractant(s) suivant(s): BE, CH, DE, FR, GB, GR, IT, LI, LU, NL, SE)

1. Composé de formule (I) ou sel de celui-ci acceptable du point de vue pharmaceutique : 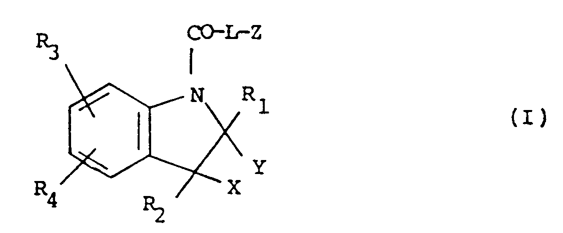 dans laquelle L est le radical NH ou l'atome d'oxygène, X et Y sont indépendamment choisis parmi un atome d'hydrogène ou un groupe alkyle en C₁₋₄ ou forment ensemble une liaison,
R₁ et R₂ sont indépendamment choisis parmi un atome d'hydrogène, un groupe alkyle en C₁₋₆ , alcényl(C₂₋₆)alkyle en C₁₋₄ ou forment ensemble un radical polyméthylène en C₂₋₄ ,
R₃ et R₄ sont indépendamment choisis parmi un atome d'hydrogène, d'halogène, un groupe CF₃, alkyle en C₁₋₆ , alcoxy en C₁₋₆ , alkylthio en C₁₋₆ , acyle en C₁₋₇ , acylamino en C₁₋₇ , alkyl(C₁₋₆)sulfonylamino, N-(alkyl(C₁₋₆)sulfonyl)-N- alkyl(C₁₋₆)amino, alkyl(C₁₋₆)sulfinyle, hydroxy, nitro ou amino, aminocarbonyle, aminosulfonyle, aminosulfonylaamino ou N-(aminosulfonyl)-alkyl(C₁₋₄)amino éventuellement N-substitué par un ou deux groupes choisis parmi les groupes alkyle en C₁₋₆ , cycloalkyle en C₃₋₈ , cycloalkyl(C₃₋₈)-alkyle en C₁₋₄ , phényle ou phénylalkyle en C₁₋₄ ou éventuellement N-disubstitué par un radical polyméthylène en C₄₋₅ ;
Z est un groupe de formule (a), (b) ou (c) : 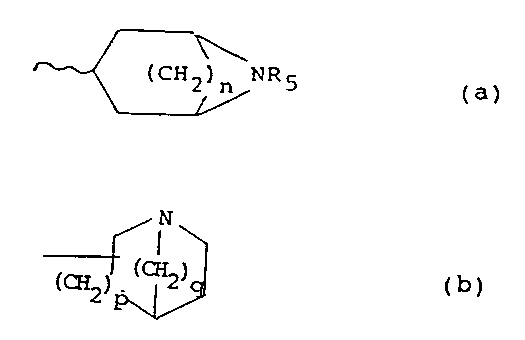 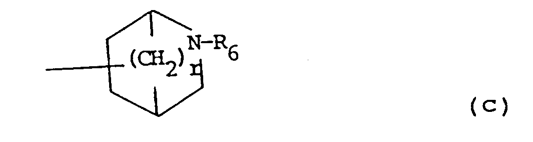 dans lesquelles n est 2 ou 3, p est 1 ou 2, q est 1 à 3, r est 1 à 3 ; et
R₅ ou R₆ est un groupe alkyle en C₁₋₇ , cycloalkyle en C₃₋₈ , cycloalkyl(C₃₋₈)alkyle en C₁₋₂ ou alcényl(C₂₋₇)alkyle en C₁₋₄ ; à condition que le composé de formule (I) soit autre que l'ester 1-azabicyclo[2,2,2]oct-3-ylique d'acide 2,3-dihydroindole-1-carboxylique.

2. Composé suivant la revendication 1, représenté par la formule (II) : 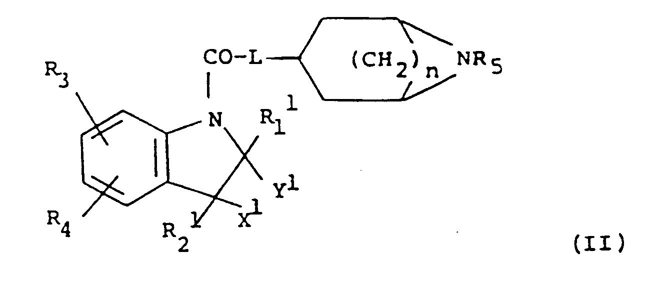 dans laquelle X¹ et Y¹ sont indépendamment un atome d'hydrogène, un groupe méthyle ou éthyle ou forment ensemble une liaison, R₁¹ et R₂¹ sont indépendamment un atome d'hydrogène, un groupe méthyle ou éthyle et les variables restantes sont telles que définies dans la revendication 1.

3. Composé suivant la revendication 2, dans lequel n est 2.

4. Composé suivant la revendication 2 ou 3, dans lequel R₅ est un groupe méthyle.

5. Composé suivant la revendication 1, représenté par la formule (III) : 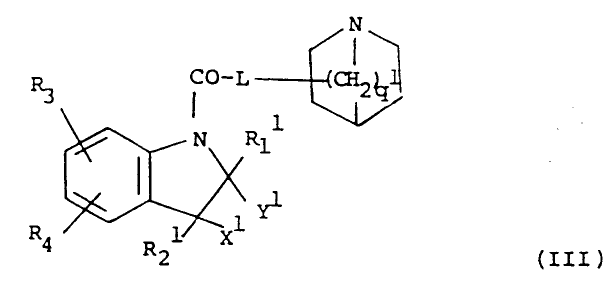 dans laquelle q¹ est 1 ou 2 et les variables restantes sont telles que définies dans les revendications 1 et 2.

6. Composé suivant la revendication 5, dans lequel q¹ est 2.

7. Composé suivant l'une quelconque des revendication 1 à 6, dans lequel l'un des R₃ et R₄ est un atome d¹hydrogène et l'autre est choisi parmi un atome d'hydrogène, un atome de chlore ou de fluor en position 5.

8. Composé suivant l'une quelconque des revendications 1 à 7, dans lequel L est un radical NH.

9. Composé suivant la revendication 1 choisi dans le groupe consistant en
endo-N-(9-méthyl-9-azabicyclo[3,3,1]-non-3-yl)-2,3-dihydroindole-1-carboxamide,
endo-N-(8-méthyl-8-azabicyclo[3,2,1]oct-3-yl)-2,3-dihydroindole-1-carboxamide,
ester endo-8-méthyl-8-azabicyclo[3,2,1]oct-3-ylique d'acide 2,3-dihydroindole carboxylique,
endo-N-(8-méthyl-8-azabicyclo[3,2,1]oct-3-yl)-2,3-dihydro-3-méthylindole-1-carboxamide,
endo-N-(8-méthyl-8-azabicyclo[3,2,1]oct-3-yl)-3-méthylindole-1-carboxamide,
endo-N-(8-éthyl-8-azabicyclo[3,2,1]oct-3-yl)-2,3-dihydroindole-1-carboxamide,
endo-N-(8-méthyl-8-azabicyclo-[3,2,1]oct-3-yl)-5-fluoro2,3-dihydroindole-1-carboxamide,
endo-N-(8-méthyl-8-azabicyclo-[3,2,1]oct-3-yl)-2,3-dihydro-5-chloroindole-1-carboxamide,
endo-N-(8-méthyl-8-azabicyclo[3,2,1]oct-3-yl)-2,3-dihydro-5-méthoxyindole-1-carboxamide,
endo-N-(8-méthyl-8-azabicyclo-[3,2,1]oct-3-yl)-indole-1-carboxamide,
N-(1-azabicyclo[2,2,2]oct-3-yl)-2,3-dihydroindole-1-carboxamide,
endo-N-(8méthyl-8-azabicyclo[3,2,1]oct-3-yl)-2,3-dihydro-3-éthylindole-1-carboxamide,
endo-N-(8-méthyl-8-azabicyclo[3,2,1]oct-3-yl)-3-éthylindole-1-carboxamide,
endo-N-(8-méthyl-8-azabicyclo[3,2,1]oct-3-yl)-2,3-dihydro-5-nitroindole-1-carboxamide,
endo-N-(8-méthyl-8-azabicyclo[3,2,1]oct-3-yl)-2,3-dihydro-6-nitroindole-1-carboxamide,
N-(1-azabicyclo[2,2,2]oct-3-yl)-2,3-dihydro-3,3-diméthylindole-1-carboxamide,
ou un sel acceptable du point de vue pharmaceutique de l'un quelconque des précédents.

10. Composé qui est endo-N-(8-méthyl-8-azabicyclo[3,2,1]oct-3-yl)-2,3-dihydro-3,3-diméthylindole-1-carboxamide ou un sel de celui-ci acceptable du point de vue pharmaceutique.

11. Procédé pour la préparation d'un composé de formule (I) suivant la revendication 1, ou d'un sel de celui-ci acceptable du point de vue pharmaceutique, qui comprend la réaction d'un composé de formule (V) : 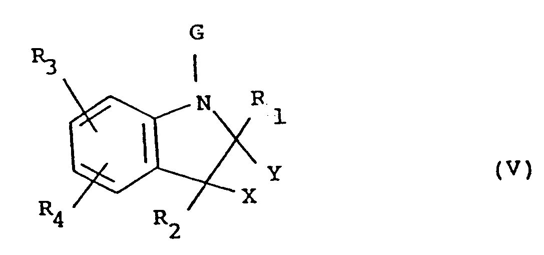 avec un composé de formule (VI) :
J-Z¹ (VI)
dans lesquelles
G est un groupe COQ₁ où Q₁ est un groupe mobile ou un atome d'hydrogène ; et lorsque G est COQ₁ , J est un groupe NH₂ ou OH ou un dérivé réactif de ceux-ci, ou, lorsque G est un atome d'hydrogène, J est un groupe contenant un groupe carbonyle activé capable de former une liaison CO-L avec le composé de formule (V) ; Z¹ est Z tel que défini ou dans lequel R₅/R₆ est remplacé par un groupe protecteur hydrogénolysable ; et les variables restantes sont telles que définies dans la revendication 1 ; et ensuite la conversion éventuelle d'un quelconque groupe R₃ et R₄ en un autre groupe R₃ et R₄ respectivement, la conversion de Z¹ , lorsqu'il est autre que Z, en un groupe Z, la conversion de X et Y en d'autres X et Y, et la formation éventuelle d'un sel acceptable du point de vue pharmaceutique du composé résultant de formule (I).

12. Composé de formule (V) suivant la revendication 11, dans lequel G est COQ₁ , à l'exclusion du chlorure de 1-(2,3-dihydro)indolylcarbonyle et à l'exclusion des composés de formule (V) dans laquelle Q₁ est un groupe méthoxy.

13. Composé choisi dans le groupe consistant en
carbamate de 1-(2,3-dihydro)-indolyltrichlorométhyle, 1-(2,3-dihydro-3-méthyl)indolyl-O-(1-succinimidyl)carbamate, 1-(2,3-dihydro-5-fluoro)indolyl-O-(1-succinimidyl)carbamate, carbamate de 1-(2,3-dihydro-5-méthoxy)indolyltrichlorométhyle, chlorure de 1-(2,3-dihydro-3-éthyl)indolylcarbonyle, carbamate de 1-(2,3-dihydro-5-nitro)indolyltrichlorométhyle, 1-[1-(2,3-dihydro-6-nitro)indolylcarbonyl]imidazole, ou chlorure de 1-(2,3-dihydro-3,3-diméthyl)indolylcarbonyle.

14. Composition pharmaceutique comprenant un composé suivant l'une quelconque des revendications 1 à 10 et un support acceptable du point de vue pharmaceutique;

15. Composé suivant l'une quelconque des revendications 1 à 10, utile en tant que substance thérapeutique active.

16. Composé suivant l'une quelconque des revendications 1 à 10, utile dans le traitement de la migraine, du névralgisme facial, et/ou de la névralgie du trijumeaux.

17. Composé suivant l'une quelconque des revendications 1 à 10, utile dans le traitement des vomissements comprenant les vomissements et les nausées induits par une irradiation ou un agent cytotoxique.

18. Composé suivant l'une quelconque des revendications 1 à 10, utile dans le traitement des troubles du système nerveux central.

19. Composé suivant l'une quelconque des revendications 1 à 10, utile dans le traitement de l'anxiété.

20. Composé suivant l'une quelconque des revendications 1 à 10, utile dans le traitement d'une psychose.

21. Composé suivant l'une quelconque des revendications 1 à 10, utile dans le traitement de l'arythmie.

22. Composé suivant l'une quelconque des revendications 1 à 10, utile dans le traitement de l'obésité.

23. Composé suivant l'une quelconque des revendications 1 à 10, utile dans le traitement du syndrome des intestins irritables.

24. Utilisation d'un composé suivant l'une quelconque des revendications 1 à 10, ou de l'ester 1-azabicyclo[2,2,2]oct-3-ylique d'acide 2,3-dihydroindole-1-carboxylique, ou d'un sel de ceux-ci acceptable du point de vue pharmaceutique, dans la préparation d'un médicament pour le traitement de la migraine, du névralgisme facial et/ou de la névralgie du trijumeaux.

25. Utilisation d'un composé suivant l'une quelconque des revendications 1 à 10, ou de l'ester 1-azabicyclo[2,2,2]oct-3-ylique d'acide 2,3-dihydroindole-1-carboxylique, ou d'un sel de ceux-ci acceptable du point de vue pharmaceutique dans la préparation d'un médicament pour le traitement des vomissements comprenant les nausées et vomissements induits par un agent cytotoxique ou une irradiation.

26. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement des troubles du système nerveux central.

27. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'anxiété.

28. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de la psychose.

29. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'arythmie.

30. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'obésité.

31. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement du syndrome des intestins irritables.

## Revendications (Revendications pour l'(les) Etat(s) contractant(s) suivant(s): ES)

1. Procédé pour la préparation d'un composé de formule (I), ou d'un sel de celui-ci acceptable du point de vue pharmaceutique : 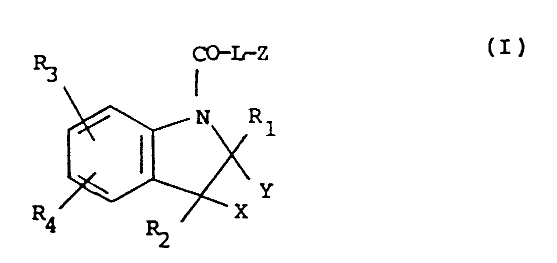 dans laquelle
L est un radical NH ou un atome d'oxygène ;
X et Y sont indépendamment choisis parmi un atome d'hydrogène ou un groupe alkyle en C₁₋₄ ou forment ensemble une liaison ;
R₁ et R₂ sont indépendamment choisis parmi un atome d'hydrogène, un groupe alkyle en C₁₋₆ , alcényl(C₂₋₆)alkyle en C₁₋₄ ou forment ensemble un radical polyméthylène en C₂₋₄ ;
R₃ et R₄ sont indépendamment choisis parmi un atome d'hydrogène, d'halogène, un groupe CF₃, alkyle en C₁₋₆ , alcoxy en C₁₋₆ , alkylthio en C₁₋₆ , acyle en C₁₋₇ , acylamino en C₁₋₇ , alkyl(C₁₋₆)sulfonylamino, N-(alkyl(C₁₋₆)sulfonyl)-N-alkylamino en C₁₋₄, alkyl(C₁₋₆)sulfinyle, hydroxy, nitro ou amino, aminocarbonyle, aminosulfonyle, aminosulfonylamino ou N-(aminosulfonyl)-alkylamino en C₁₋₄ , éventuellement N-substitué par un ou deux groupes choisis parmi les groupes alkyle en C₁₋₆ , cycloalkyle en C₃₋₈ , cycloalkyl(C₃₋₈)alkyle en C₁₋₄ , phényle ou phénylalkyle en C₁₋₄ ou éventuellement N-disubstitué par un radical polyméthylène en C₄₋₅ ;
Z est un groupe de formule (a), (b) ou (c) : 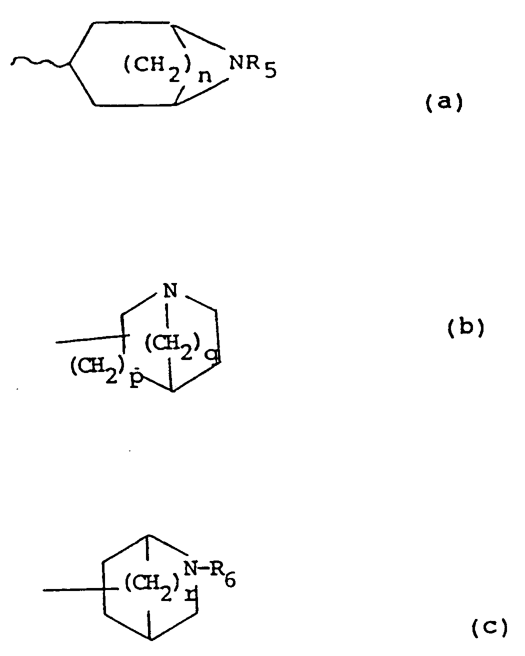 dans laquelle n est 2 ou 3, p est 1 ou 2, q est 1 à 3, r est 1 à 3 ; et
R₅ ou R₆ est un groupe alkyle en C₁₋₇ , cycloalkyle en C₃₋₈ , cycloalkyl(C₃₋₈)alkyle en C₁₋₂ ou alcényl(C₂₋₇)alkyle en C₁₋₄ , à condition que le composé de formule (I) soit autre que l'ester 1-azabicyclo[2,2,2]oct-3-ylique d'acide 2,3-dihydroindole-1-carboxylique ;
lequel procédé comprend la réaction d'un composé de formule (V) : 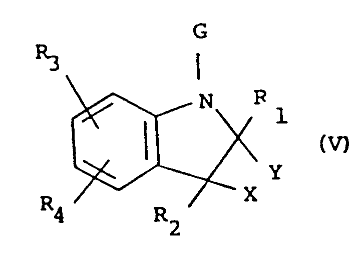 avec un composé de formule (VI) :
J-Z¹ (VI)
dans lesquelles
G est un groupe COQ₁ dans lequel Q₁ est un groupe mobile ou un atome d'hydrogène, et, lorsque G est un groupe COQ₁ , J est NH₂ ou OH ou un dérivé réactif de ceux-ci ou, lorsque G est un atome d'hydrogène, J est un groupe contenant un groupe carbonyle activé capable de former une liaison CO-L avec le composé de formule (V) ; Z¹ est un groupe Z tel que défini ou dans lequel R₅/R₆ est remplacé par un groupe protecteur hydrogénolysable ; et les variables restantes sont telles que définies dans la revendication 1, et ensuite, la conversion éventuelle d'un quelconque groupe R₃ et R₄ en un autre groupe R₃ et R₄ respectivement, la conversion de Z¹ lorsqu'il est autre que Z en un groupe Z ; la conversion de X et de Y en d'autres X et Y et la formation éventuelle d'un sel acceptable du point de vue pharmaceutique du composé résultant de formule (I).

2. Procédé pour la préparation d'un composé suivant la revendication 1, représenté par la formule (II) : 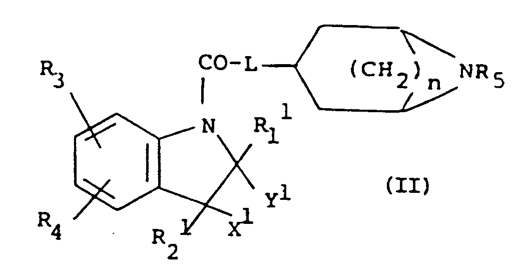 dans laquelle X¹ et Y¹ sont indépendamment un atome d'hydrogène, un groupe méthyle ou éthyle, ou forment ensemble une liaison, R₁¹ et R₂¹ sont indépendamment un atome d'hydrogène, un groupe méthyle ou éthyle et les variables restantes sont telles que définies dans la revendication 1.

3. Procédé suivant la revendication 2, dans lequel n est 2.

4. Procédé suivant la revendication 2 ou 3, dans lequel R₅ est un groupe méthyle.

5. Procédé pour la préparation d'un composé suivant la revendication 1, représenté par la formule (III) : 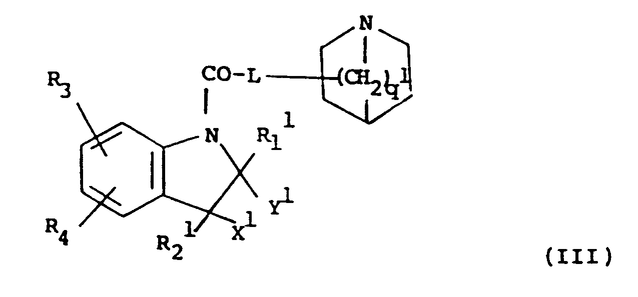 dans laquelle q¹ est 1 ou 2 et les variables restantes sont telles que définies dans les revendications 1 et 2.

6. Procédé suivant la revendication 5, dans lequel q¹ est 2.

7. Procédé suivant l'une quelconque des revendications 1 à 6, dans lequel l'un des R₃ et R₄ est un atome d'hydrogène et l'autre est choisi parmi un atome d'hydrogène, un atome de chlore ou de fluor en position 5.

8. Procédé suivant l'une quelconque des revendications 1 à 7, dans lequel L est le radical NH.

9. Procédé suivant la revendication 1, pour la préparation des composés suivants :
endo-N-(9-méthyl-9-azabicyclo[3,3,1]-non-3-yl)-2,3-dihydroindole-1-carboxamide,
endo-N-(8-méthyl-8-azabicyclo[3,2,1]oct-3-yl)-2,3-dihydroindole-1-carboxamide,
ester endo-8-méthyl-8-azabicyclo[3,2,1]oct-3-ylique d'acide 2,3-dihydroindole carboxylique,
endo-N-(8-méthyl-8-azabicyclo[3,2,1]oct-3-yl)-2,3-dihydro-3-méthylindole-1-carboxamide,
endo-N-(8-méthyl-8-azabicyclo[3,2,1]oct-3-yl)-3-méthylindole-1-carboxamide,
endo-N-(8-éthyl-8-azabicyclo[3,2,1]oct-3-yl)-2,3-dihydroindole-1-carboxamide,
endo-N-(8-méthyl-8-azabicyclo-[3,2,1]oct-3-yl)-5-fluoro-2,3-dihydroindole-1-carboxamide,
endo-N-(8-méthyl-8-azabicyclo-[3,2,1]oct-3-yl)-2,3-dihydro-5-chloroindole-1-carboxamide,
endo-N-(8-méthyl-8-azabicyclo[3,2,1]oct-3-yl)-2,3-dihydro-5-méthoxyindole-1-carboxamide,
endo-N-(8-méthyl-8-azabicyclo-[3,2,1]oct-3-yl)-indole-1-carboxamide,
N-(1-azabicyclo[2,2,2]oct-3-yl)-2,3-dihydroindole-1-carboxamide,
endo-N-(8méthyl-8-azabicyclo[3,2,1]oct-3-yl)-2,3-dihydro-3-éthylindole-1-carboxamide,
endo-N-(8-méthyl-8-azabicyclo[3,2,1]oct-3-yl)-3-éthylindole-1-carboxamide,
endo-N-(8-méthyl-8-azabicyclo[3,2,1]oct-3-yl)-2,3-dihydro-5-nitroindole-1-carboxamide,
endo-N-(8-méthyl-8-azabicyclo[3,2,1]oct-3-yl)-2,3-dihydro-6-nitroindole-1-carboxamide,
N-(1-azabicyclo[2,2,2]oct-3-yl)-2,3-dihydro-3,3-diméthylindole-1-carboxamide,
ou un sel acceptable du point de vue pharmaceutique de l'un quelconque des précédents.

10. Procédé suivant la revendication 1, pour la préparation de l'endo-N-(8-méthyl-8-azabicyclo[3,2,1]oct-3-yl)-2,3-dihydro-3,3-diméthylindole-1-carboxamide ou d'un sel de celui-ci acceptable du point de vue pharmaceutique.

11. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement de la migraine, du névralgisme facial et/ou de la névralgie du trijumeaux.

12. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement des vomissements comprenant les nausées et vomissements induits par un agent cytotoxique ou une irradiation.

13. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement des troubles du système nerveux.

14. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement de l'anxiété.

15. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement de la psychose.

16. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement de l'arythmie.

17. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement de l'obésité.

18. Utilisation d'un composé de formule (I) suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique dans la préparation d'un médicament pour le traitement du syndrome des intestins irritables.

## Revendications (Revendications pour l'(les) Etat(s) contractant(s) suivant(s): GR)

1. Composé de formule (I) ou sel de celui-ci acceptable du point de vue pharmaceutique : 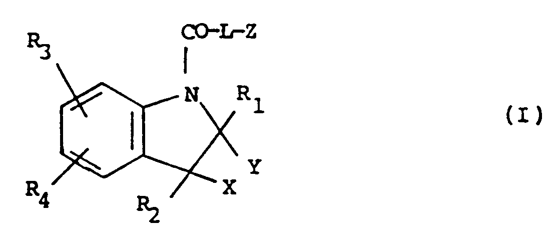 dans laquelle L est le radical NH ou l'atome d'oxygène, X et Y sont indépendamment choisis parmi un atome d'hydrogène ou un groupe alkyle en C₁₋₄ ou forment ensemble une liaison,
R₁ et R₂ sont indépendamment choisis parmi un atome d'hydrogène, un groupe alkyle en C₁₋₆ , alcényl(C₂₋₆)alkyle en C₁₋₄ ou forment ensemble un radical polyméthylène en C₂₋₄ ,
R₃ et R₄ sont indépendamment choisis parmi un atome d'hydrogène, d'halogène, un groupe CF₃, alkyle en C₁₋₆ , alcoxy en C₁₋₆ , alkylthio en C₁₋₆ , acyle en C₁₋₇ , acylamino en C₁₋₇ , alkyl(C₁₋₆)sulfonylamino, N-(alkyl(C₁₋₆)sulfonyl)-N- alkyl(C₁₋₆)amino, alkyl(C₁₋₆)sulfinyle, hydroxy, nitro ou amino, aminocarbonyle, aminosulfonyle, aminosulfonylaamino ou N-(aminosulfonyl)-alkyl(C₁₋₄)amino éventuellement N-substitué par un ou deux groupes choisis parmi les groupes alkyle en C₁₋₆ , cycloalkyle en C₃₋₈ , cycloalkyl(C₃₋₈)alkyle en C₁₋₄ , phényle ou phénylalkyle en C₁₋₄ ou éventuellement N-disubstitué par un radical polyméthylène en C₄₋₅ ;
Z est un groupe de formule (a), (b) ou (c) : 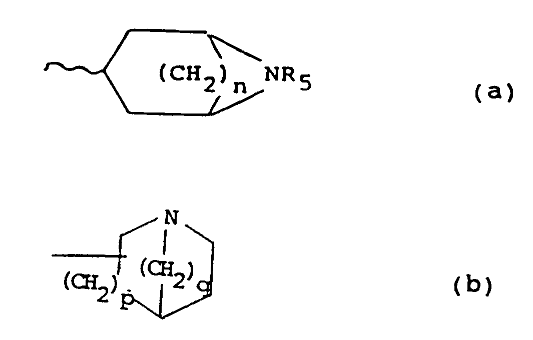 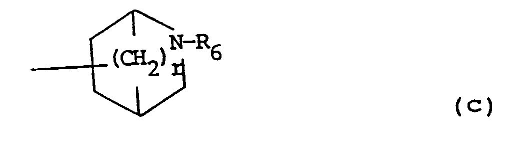 dans lesquelles n est 2 ou 3, p est 1 ou 2, q est 1 à 3, r est 1 à 3 ; et
R₅ ou R₆ est un groupe alkyle en C₁₋₇ , cycloalkyle en C₃₋₈ , cycloalkyl(C₃₋₈)alkyle en C₁₋₂ ou alcényl(C₂₋₇)alkyle en C₁₋₄ ; à condition que le composé de formule (I) soit autre que l'ester 1-azabicyclo[2,2,2]oct-3-ylique d'acide 2,3-dihydroindole-1-carboxylique.

2. Composé suivant la revendication 1, représenté par la formule (II) : 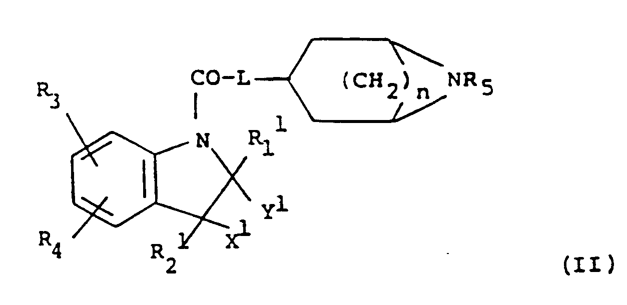 dans laquelle X¹ et Y¹ sont indépendamment un atome d'hydrogène, un groupe méthyle ou éthyle ou forment ensemble une liaison, R₁¹ et R₂¹ sont indépendamment un atome d'hydrogène, un groupe méthyle ou éthyle et les variables restantes sont telles que définies dans la revendication 1.

3. Composé suivant la revendication 2, dans lequel n est 2.

4. Composé suivant la revendication 2 ou 3, dans lequel R₅ est un groupe méthyle.

5. Composé suivant la revendication 1, représenté par la formule (III) : 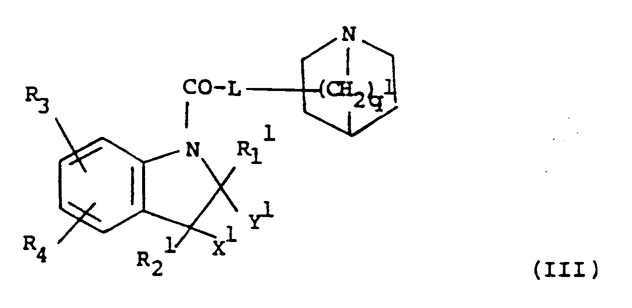 dans laquelle q¹ est 1 ou 2 et les variables restantes sont telles que définies dans les revendications 1 et 2.

6. Composé suivant la revendication 5, dans lequel q¹ est 2.

7. Composé suivant l'une quelconque des revendication 1 à 6, dans lequel l'un des R₃ et R₄ est un atome d'hydrogène et l'autre est choisi parmi un atome d'hydrogène, un atome de chlore ou de fluor en position 5.

8. Composé suivant l'une quelconque des revendications 1 à 7, dans lequel L est un radical NH.

9. Composé suivant la revendication 1 choisi dans le groupe consistant en
endo-N-(9-méthyl-9-azabicyclo[3,3,1]-non-3-yl)-2,3-dihydroindole-1-carboxamide,
endo-N-(8-méthyl-8-azabicyclo[3,2,1]oct-3-yl)-2,3-dihydroindole-1-carboxamide,
ester endo-8-méthyl-8-azabicyclo[3,2,1]oct-3-ylique d'acide 2,3-dihydroindole carboxylique,
endo-N-(8-méthyl-8-azabicyclo[3,2,1]oct-3-yl)-2,3-dihydro-3-méthylindole-1-carboxamide,
endo-N-(8-méthyl-8-azabicyclo[3,2,1]oct-3-yl)-3-méthylindole-1-carboxamide,
endo-N-(8-éthyl-8-azabicyclo[3,2,1]oct-3-yl)-2,3-dihydroindole-1-carboxamide,
endo-N-(8-méthyl-8-azabicyclo-[3,2,1]oct-3-yl)-5-fluoro-2,3-dihydroindole-1-carboxamide,
endo-N-(8-méthyl-8-azabicyclo-[3,2,1]oct-3-yl)-2,3-dihydro-5-chloroindole-1-carboxamide,
endo-N-(8-méthyl-8-azabicyclo[3,2,1]oct-3-yl)-2,3-dihydro-5-méthoxyindole-1-carboxamide,
endo-N-(8-méthyl-8-azabicyclo-[3,2,1]oct-3-yl)-indole-1-carboxamide,
N-(1-azabicyclo[2,2,2]oct-3-yl)-2,3-dihydroindole-1-carboxamide,
endo-N-(8méthyl-8-azabicyclo[3,2,1]oct-3-yl)-2,3-dihydro-3-éthylindole-1-carboxamide,
endo-N-(8-méthyl-8-azabicyclo[3,2,1]oct-3-yl)-3-éthylindole-1-carboxamide,
endo-N-(8-méthyl-8-azabicyclo[3,2,1]oct-3-yl)-2,3-dihydro-5-nitroindole-1-carboxamide,
endo-N-(8-méthyl-8-azabicyclo[3,2,1]oct-3-yl)-2,3-dihydro-6-nitroindole-1-carboxamide,
N-(1-azabicyclo[2,2,2]oct-3-yl)-2,3-dihydro-3,3-diméthylindole-1-carboxamide,
ou un sel acceptable du point de vue pharmaceutique de l'un quelconque des précédents.

10. Composé qui est l'endo-N-(8-méthyl-8-azabicyclo[3,2,1]oct-3-yl)-2,3-dihydro-3,3-diméthylindole-1-carboxamide ou un sel de celui-ci acceptable du point de vue pharmaceutique.

11. Procédé pour la préparation d'un composé de formule (I) suivant la revendication 1, ou d'un sel de celui-ci acceptable du point de vue pharmaceutique, qui comprend la réaction d'un composé de formule (V) : 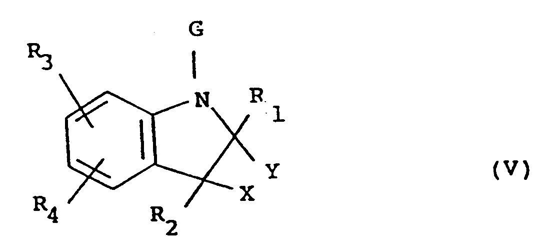 avec un composé de formule (VI) :
J-Z¹ (VI)
dans lesquelles
G est un groupe COQ₁ où Q₁ est un groupe mobile ou un atome d'hydrogène ; et lorsque G est COQ₁ , J est un groupe NH₂ ou OH ou un dérivé réactif de ceux-ci, ou, lorsque G est un atome d'hydrogène, J est un groupe contenant un groupe carbonyle activé capable de former une liaison CO-L avec le composé de formule (V) ; Z¹ est Z tel que défini ou dans lequel R₅/R₆ est remplacé par un groupe protecteur hydrogénolysable ; et les variables restantes sont telles que définies dans la revendication 1 ; et ensuite la conversion éventuelle d'un quelconque groupe R₃ et R₄ en un autre groupe R₃ et R₄ respectivement, la conversion de Z¹ , lorsqu'il est autre que Z, en un groupe Z, la conversion de X et Y en d'autres X et Y, et la formation éventuelle d'un sel acceptable du point de vue pharmaceutique du composé résultant de formule (I).

12. Composé de formule (V) suivant la revendication 11, dans lequel G est COQ₁ , à l'exclusion du chlorure de 1-(2,3-dihydro)indolylcarbonyle et à l'exclusion des composés de formule (V) dans laquelle Q₁ est un groupe méthoxy.

13. Composé choisi dans le groupe consistant en
carbamate de 1-(2,3-dihydro)-indolyltrichlorométhyle, 1-(2,3-dihydro-3-méthyl)indolyl-O-(1-succinimidyl)carbamate, 1-(2,3-dihydro-5-fluoro)indolyl-O-(1-succinimidyl)carbamate, carbamate de 1-(2,3-dihydro-5-méthoxy)indolyltrichlorométhyle, chlorure de 1-(2,3-dihydro-3-éthyl)indolylcarbonyle, carbamate de 1-(2,3-dihydro-5-nitro)indolyltrichlorométhyle, 1-[1-(2,3-dihydro-6-nitro)indolylcarbonyl]imidazole, ou chlorure de 1-(2,3-dihydro-3,3-diméthyl)indolylcarbonyle.

14. Utilisation d'un composé suivant l'une quelconque des revendications 1 à 10, ou de l'ester 1-azabicyclo[2,2,2]oct-3-ylique d'acide 2,3-dihydroindole-1-carboxylique, ou d'un sel de ceux-ci acceptable du point de vue pharmaceutique, dans la préparation d'un médicament pour le traitement de la migraine, du névralgisme facial et/ou de la névralgie du trijumeaux.

15. Utilisation d'un composé suivant l'une quelconque des revendications 1 à 10, ou de l'ester 1-azabicyclo[2,2,2]oct-3-ylique d'acide 2,3-dihydroindole-1-carboxylique, ou d'un sel de ceux-ci acceptable du point de vue pharmaceutique dans la préparation d'un médicament pour le traitement des vomissements comprenant les nausées et vomissements induits par un agent cytotoxique ou une irradiation.

16. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement des troubles du système nerveux central.

17. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'anxiété.

18. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de la psychose.

19. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'arythmie.

20. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement de l'obésité.

21. Utilisation d'un composé suivant l'une quelconque des revendications 1 à 10, ou de l'ester 1-azabicyclo[2,2,2]oct-3-ylique d'acide 2,3-dihydroindole-1-carboxylique ou d'un sel de ceux-ci acceptable du point de vue pharmaceutique, dans la préparation d'un médicament pour le traitement du syndrome des intestins irritables.
